# EUROPEAN PATENT APPLICATION

(11) **EP 4 470 562 A1**
(43) Date of publication of application: **04.12.2024**
(21) Application number: 23747044.8
(22) Date of filing: 26.01.2023
(51) Int. Cl.: A61K 39/395, A61P 35/00, A61K 31/337

(54) **ANTI-PD-L1 ANTIBODY-CONTAINING PHARMACEUTICAL COMPOSITION USED IN COMBINATION WITH ANTI-VEGF ANTIBODIES AND PACLITAXEL**

(30) Priority: 27.01.2022 WO PCT/JP2022/003166
(71) Applicant: CHUGAI SEIYAKU KABUSHIKI KAISHA, Tokyo 115-8543 (JP); GENENTECH, INC., South San Francisco, CA 94080 (US)
(72) Inventor: TAKAHASHI, Koji, Tokyo 103-8324 (JP); AKAMATSU, Ayumi, Tokyo 103-8324 (JP); TAKEMOTO, Shinya, Tokyo 103-8324 (JP); IWAI, Toshiki, Kamakura-shi, Kanagawa 247-8530 (JP); HARA, Fumikata, Tokyo 135-8550 (JP); YONEMORI, Kan, Tokyo 104-0045 (JP); SHIEN, Tadahiko, Okayama-shi, Okayama 700-8558 (JP)
(74) Representative: Vossius & Partner Patentanwälte Rechtsanwälte mbB
(86) International application number: PCT/JP2023/002476
(87) International publication number: WO 2023/145834

(57) **Abstract**

The present disclosure relates to a pharmaceutical composition containing paclitaxel, an anti-VEGF antibody and an anti-PD-L1 antibody.

## Description

### TECHNICAL FIELD

The present disclosure relates to a pharmaceutical composition comprising an anti-PD-Ll antibody as an active ingredient, characterized by being used in combination with a VEGF signal inhibitor (more specifically, an anti-VEGF antibody) and paclitaxel.

### BACKGROUND ART

Immune checkpoints have functions of suppressing excessive immune reactions and suppressing occurrence of autoimmune disease, etc. As the immune checkpoint proteins that pertain to this mechanism, PD-1 on T cells, etc., that respond to PD-L1 ligands on the surface of tumor cells and immune cells, etc., have been known. Antibodies against these proteins, for example, anti-PD-L1 antibody, are immune checkpoint inhibitors. By administering such immune checkpoint inhibitors, immunosuppression of T cells is released and anti-tumor immune response is enhanced.

Human vascular endothelial growth factor (VEGF/VEGF-A) is involved in restriction of normal and abnormal angiogenesis, and neovascularization, associated with tumors and intraocular disorders. VEGF is a homodimeric glycoprotein that has been isolated from multiple sources. VEGF exhibits mitogenic activity on endothelial cells. VEGF has important regulatory functions in the formation of new blood vessels during fetal angiogenesis and in adult angiogenesis. As drugs called angiogenesis inhibitors (VEGF inhibitors), three products have been approved for manufacturing and marketing in Japan as of October 2018. The three products are bevacizumab (trade name Avastin), ramucirumab (trade name Cyramza) and aflibercept beta (trade name Zaltrap).

Paclitaxel is one of the mitotic inhibitors belonging to the taxane family, isolated from the bark of the Pacific yew (Taxus brevifolia) and named "taxol". Paclitaxel suppresses microtubule dynamics by stabilizing microtubules, thereby preventing normal progression of cell division. For the purpose of mainly alleviating side effects of paclitaxel, development of anticancer agents of derivatives of paclitaxel and drug delivery system (DDS) formulations have been developed, and in Japan, albumin-bound paclitaxel (nab-paclitaxel; ABRAXANE) has been launched and polyglutamated paclitaxel (paclitaxel poliglumex; OPAXIO) is known to be in phase III clinical trials.

A variety of cancer treatments with a combination of a PD-1-binding antagonist, which is one of the immune checkpoint inhibitors, and chemotherapy and further combining them with a VEGF antagonist are known (Patent Documents 1 and 2), and among them, in the treatment of breast cancer, combination therapy with a taxane-based chemotherapeutic agent as a chemotherapeutic agent is mainly known (Patent Documents 3 to 6).

When divided by breast cancer subtype, in advanced recurrent triple-negative breast cancer, a combination therapy of Pembrolizumab which is an anti-PD-1 antibody as a PD-1 axis inhibitor, and a chemotherapeutic agent such as nab-paclitaxel, paclitaxel, or gemcitabine + carboplatin was developed (Non-Patent Documents 2 and 4), and a phase III clinical trial of atezolizumab which is an anti-PD-Ll antibody, and a taxane-based chemotherapeutic agent in advanced recurrent triple-negative breast cancer was carried out, but in the case of atezolizumab, clinical effects of using nab-paclitaxel and paclitaxel as a taxane-based chemotherapeutic agent are different, and desirable results could not be obtained in combination with paclitaxel (Non-Patent Document 3). Currently, a phase II clinical trial of the primary treatment of advanced recurrent triple-negative breast cancer is enrolling with three agents of atezolizumab, paclitaxel and bevacizumab in combination (Non-Patent Document 7).

On the other hand, as a treatment for hormone receptor-positive (at least one of estrogen receptor and progesterone receptor is positive) HER2-negative breast cancer, combination therapy with pembrolizumab, which is an anti-PD-1 antibody, and eribulin, which is a microtubule inhibitor, has been tried, but elongation of PFS could not be obtained (Non-Patent Document 1). In addition, a phase II clinical trial of combination therapy with nivolumab, which is an anti-PD-1 antibody, and paclitaxel and bevacizumab was reported in 2020 (Non-Patent Document 5), but thereafter, no clinical trial intended for development has been published as of January 2022.

That is, with regard to anti-PD-Ll antibody therapy and combination therapy with an anti-PD-Ll antibody and a taxane-based chemotherapeutic agent for hormone receptor-positive HER2-negative breast cancer, more effective treatment method is still being sought.

### PRIOR ART DOCUMENTS

### PATENT DOCUMENTS

Patent Document 1: JP 2015-519372A
Patent Document 2: Japanese Patent No. 6,509,935
Patent Document 3: JP 2017-501155A
Patent Document 4: JP 2018-522851A
Patent Document 5: WO2020061349
Patent Document 6: JP 2018-501218S

### NON-PATENT DOCUMENTS

Non-Patent Document 1: ASCO2019, Abstract 1004.
Non-Patent Document 2: The Lancet, 396, ISSUE10265, P1817-1828, DEC, 05, 2020
Non-Patent Document 3: ESMO Open. 2020; 5(6): e001112.
Non-Patent Document 4: Cancer Immunology, Immunotherapy vol. 70, pp. 607-617 (2021)
Non-Patent Document 5: AACR; Cancer Res 2020; 80 (4 Suppl) Abstract nrPD 1-03
Non-Patent Document 6: JAMA Oncol. 2020 May; 6(5): 676-684.
Non-Patent Document 7: NCT04408118, https://clinicaltrials.gov/ct2/show/NCT04408118?term=NCT04408118&draw=2&rank =1

### SUMMARY OF THE INVENTION

### PROBLEMS TO BE SOLVED BY THE INVENTION

The present disclosure provides a combination of a novel anti-cancer agent and immunotherapy for the treatment of estrogen receptor-positive HER2-negative breast cancer. In addition, the present disclosure provides a combination of a novel anti-cancer agent and immunotherapy for the treatment of hormone receptor-positive HER2-negative breast cancer.

### MEANS TO SOLVE THE PROBLEMS

As a result of intensive research, the present disclosing person has found that anti-PD-L1 antibody therapy is effective for treating estrogen receptor-positive HER2-negative breast cancer or hormone receptor-positive HER2-negative breast cancer and a combination of an anti-PD-Ll antibody with an anti-VEGF antibody and paclitaxel exhibits a synergistic effect, whereby accomplished the present disclosure.

The present disclosure includes the following embodiments.
[1] A pharmaceutical composition which is a composition for use in combination with paclitaxel and an anti-VEGF antibody to treat estrogen receptor-positive HER2-negative breast cancer or to delay progression of the cancer in an individual, which contains an anti-PD-Ll antibody as an effective ingredient.
[2] The pharmaceutical composition described in [1], wherein the anti-PD-L1 antibody is atezolizumab.
[3] The pharmaceutical composition described in any of [1] to [2], wherein the anti-VEGF antibody is bevacizumab, ramucirumab or aflibercept beta.
[4] The pharmaceutical composition described in any of [1] to [3], wherein the anti-VEGF antibody is bevacizumab.
[5] The pharmaceutical composition described in any of [1] to [4], wherein the breast cancer is hormone receptor-positive (at least one of estrogen receptor and progesterone receptor is positive) and HER2-negative breast cancer.
   [5-1] The composition described in any of [1] to [5], wherein use of the composition comprises administering atezolizumab at a dose of 840 mg/body once every two weeks.
   [5-2] The composition described in any of [1] to [5-1], wherein use of the composition comprises administering paclitaxel at a dose of 90 mg/m² (body surface area) once a week (28 days as one cycle and administered on days 1, 8 and 15), administering atezolizumab at a dose of 840 mg/body once every two weeks, and administering bevacizumab at a dose of 10 mg/kg (body weight) every two weeks.
   [5-3] The composition described in any of [1] to [5], [5-1] and [5-2], wherein, by the use of the composition, a median value of progression-free survival period of the individual is elongated as compared with an individual having hormone receptor-positive HER2-negative breast cancer treated with paclitaxel and bevacizumab.
   [5-4] The composition described in any of [1] to [5] and [5-1] to [5-3], wherein, by the use of the composition, a median value of progression-free survival period of the individual is elongated at least about 3 months, preferably about 4 months, and more preferably about 5 months as compared with an individual having hormone receptor-positive HER2-negative breast cancer treated with paclitaxel and bevacizumab.
[6] A method for treating estrogen receptor-positive HER2-negative breast cancer or delaying progression of the cancer, which comprises administering an anti-PD-Ll antibody, an anti-VEGF antibody and paclitaxel in combination.
[7] The method described in [6], wherein the anti-PD-Ll antibody is atezolizumab.
[8] The method described in any of [6] to [7], wherein the anti-VEGF antibody is bevacizumab, ramucirumab or aflibercept beta.
[9] The method described in any of [6] to [8], wherein the anti-VEGF antibody is bevacizumab.
[10] The method described in any of [6] to [9], wherein the breast cancer is hormone receptor-positive HER2-negative breast cancer.
   [10-1] The method described in any of [6] to [10], wherein the method comprises administering atezolizumab at a dose of 840 mg/body once every two weeks.
   [10-2] The method described in any of [6] to [10] and [10-1], wherein the method comprises administering paclitaxel at a dose of 90 mg/m² (body surface area) once a week (28 days as one cycle and administered on days 1, 8 and 15), administering atezolizumab at a dose of 840 mg/body once every two weeks, and administering bevacizumab at a dose of 10 mg/kg (body weight) every two weeks.
   [10-3] The method described in any of [6] to [10], [10-1] and [10-2], wherein a median value of progression-free survival period of the individual is elongated by the method as compared with an individual having hormone receptor-positive HER2-negative breast cancer treated with paclitaxel and bevacizumab.
   [10-4] The method described in any of [6] to [10] and [10-1] to [10-3], wherein a median value of progression-free survival period of the individual is elongated by the method at least about 3 months, preferably about 4 months, and more preferably about 5 months as compared with an individual having hormone receptor-positive HER2-negative breast cancer treated with paclitaxel and bevacizumab.
[11] A medicament for treating or delaying progression of estrogen receptor-positive HER2-negative breast cancer in an individual for use in combination with paclitaxel and bevacizumab, wherein the medicament comprises atezolizumab.
[12] The medicament described in [11], wherein the breast cancer is hormone receptor-positive HER2-negative breast cancer.
   [12-1] The medicament described in any of [11] to [12], wherein the medicament comprises atezolizumab administered at a dose of 840 mg/body once every two weeks.
   [12-2] The medicament described in any of [11] to [12] and [12-1], wherein the medicament comprises paclitaxel administered at a dose of 90 mg/m² (body surface area) once a week (28 days as one cycle and administered on days 1, 8 and 15), atezolizumab administered at a dose of 840 mg/body once every two weeks, and bevacizumab administered at a dose of 10 mg/kg (body weight) every two weeks.
   [12-3] The medicament described in any of [11] to [12], [12-1] and [12-2], wherein a median value of progression-free survival period of the individual is elongated by the medicament as compared with an individual having hormone receptor-positive HER2-negative breast cancer treated with paclitaxel and bevacizumab.
   [12-4] The medicament described in any of [11] to [12] and [12-1] to [12-3], wherein a median value of progression-free survival period of the individual is elongated by the medicament at least about 3 months, preferably about 4 months, and more preferably about 5 months as compared with an individual having hormone receptor-positive HER2-negative breast cancer treated with paclitaxel and bevacizumab.
[13] A method for treating estrogen receptor-positive HER2-negative breast cancer which comprises using atezolizumab, anti-VEGF antibody and paclitaxel in combination.
[14] The method described in [13], wherein the breast cancer is hormone receptor-positive HER2-negative breast cancer.
   [14-1] The method described in any of [13] to [14], wherein the method comprises administering atezolizumab at a dose of 840 mg/body once every two weeks.
   [14-2] The method described in any of [13] to [14] and [14-1], wherein the method comprises administering paclitaxel at a dose of 90 mg/m² (body surface area) once a week (28 days as one cycle and administered on days 1, 8 and 15), administering atezolizumab at a dose of 840 mg/body once every two weeks, and administering bevacizumab at a dose of 10 mg/kg (body weight) every two weeks.
   [14-3] The method described in any of [13] to [14], [14-1] and [14-2], wherein, by the method, a median value of progression-free survival period of the individual is elongated as compared with an individual having hormone receptor-positive HER2-negative breast cancer treated with paclitaxel and bevacizumab.
   [14-4] The method described in any of [13] to [14] and [14-1] to [14-3], wherein, by the method, a median value of progression-free survival period of the individual is elongated at least about 3 months, preferably about 4 months, and more preferably about 5 months as compared with an individual having hormone receptor-positive HER2-negative breast cancer treated with paclitaxel and bevacizumab.
[15] A composition for treating estrogen receptor-positive HER2-negative breast cancer or delaying progression of the cancer in an individual comprising an effective amount of an anti-PD-L1 antibody, and is a composition for use in combination with an anti-VEGF antibody and paclitaxel,
   wherein the anti-PD-Ll antibody comprises a heavy chain variable region and a light chain variable region,
      (a) the heavy chain variable region comprises HVR-H1, HVR-H2 and HVR-H3,
         (i) HVR-H1 comprises an amino acid sequence of SEQ ID NO: 1,
         (ii) HVR-H2 comprises an amino acid sequence of SEQ ID NO: 2, and
         (iii) HVR-H3 comprises an amino acid sequence of SEQ ID NO: 3,
      (b) the light chain variable region comprises HVR-L1, HVR-L2 and HVR-L3,
         (iv) HVR-L1 comprises an amino acid sequence of SEQ ID NO: 4,
         (v) HVR-L2 comprises an amino acid sequence of SEQ ID NO: 5, and
         (vi) HVR-L3 comprises an amino acid sequence of SEQ ID NO: 6,
   the anti-VEGF antibody comprises a heavy chain variable region and a light chain variable region,
      (a) the heavy chain variable region comprises CDRH1, CDRH2 and CDRH3,
         (i) CDRH1 comprises an amino acid sequence of SEQ ID NO: 11,
         (ii) CDRH2 comprises an amino acid sequence of SEQ ID NO: 12, and
         (iii) CDRH3 comprises an amino acid sequence of SEQ ID NO: 13,
      (b) the light chain variable region comprises CDRL1, CDRL2 and CDRL3,
         (iv) CDRL1 comprises an amino acid sequence of SEQ ID NO: 14,
         (v) CDRL2 comprises an amino acid sequence of SEQ ID NO: 15, and
         (vi) CDRL3 comprises an amino acid sequence of SEQ ID NO: 16.
[16] The composition described in [15], wherein the anti-PD-Ll antibody is a monoclonal antibody.
[17] The composition described in any of [15] to [16], wherein the anti-PD-L1 antibody is a humanized antibody.
[18] The composition described in any of [15] to [17], wherein the anti-PD-Ll antibody comprises a heavy chain variable region and a light chain variable region, and
   (a) an amino acid sequence of the heavy chain variable region has sequence identity of at least 90% to an amino acid sequence of the heavy chain variable region of SEQ ID NO: 7, and
   (b) an amino acid sequence of the light chain variable region has sequence identity of at least 90% to an amino acid sequence of the light chain variable region of SEQ ID NO: 8.
[19] The composition described in any one of [15] to [18], wherein the anti-PD-L1 antibody comprises a heavy chain variable region and a light chain variable region,
   (a) an amino acid sequence of the heavy chain variable region has sequence identity of at least 95% to an amino acid sequence of the heavy chain variable region of SEQ ID NO: 7, and
   (b) an amino acid sequence of the light chain variable region has sequence identity of at least 95% to an amino acid sequence of the light chain variable region of SEQ ID NO: 8.
[20] The composition described in any one of [15] to [19], wherein the anti-PD-Ll antibody comprises a heavy chain variable region and a light chain variable region,
   (a) an amino acid sequence of the heavy chain variable region has sequence identity of at least 99% to an amino acid sequence of the heavy chain variable region of SEQ ID NO: 7, and
   (b) an amino acid sequence of the light chain variable region has sequence identity of at least 99% to an amino acid sequence of the light chain variable region of SEQ ID NO: 8.
[21] The composition described in any one of [15] to [20], wherein the anti-PD-Ll antibody comprises a heavy chain variable region and a light chain variable region,
   (a) an amino acid sequence of the heavy chain variable region has sequence identity of at least 99% to an amino acid sequence of the heavy chain variable region of SEQ ID NO: 7, and
   (b) an amino acid sequence of the light chain variable region comprises an amino acid sequence of the light chain variable region of SEQ ID NO: 8.
[22] The composition described in any of [15] to [21], wherein the anti-PD-Ll antibody further comprises a human IgG1 constant region.
[23] The composition described in [22], wherein the anti-PD-Ll antibody comprises an effectorless Fc mutation, and the effectorless Fc mutation is N297A.
[24] The composition described in [15], wherein the anti-PD-Ll antibody is 10F.9G2.
[25] The composition described in any of [1] to [5] and [15] to [24], wherein the anti-VEGF antibody binds to the same epitope as a monoclonal anti-VEGF antibody A4.6.1 produced by hybridoma ATCC HB 10709.
[26] The composition described in any one of [15] to [24], wherein the anti-VEGF antibody is a humanized antibody.
[27] The composition described in any one of [15] to [24], wherein the anti-VEGF antibody is bevacizumab.
[28] The composition described in any of [15] to [27], wherein the anti-VEGF antibody comprises a heavy chain variable region and a light chain variable region,
   (a) the heavy chain variable region comprises CDRH1, CDRH2 and CDRH3,
      (i) CDRH1 comprises an amino acid sequence of SEQ ID NO: 11,
      (ii) CDRH2 comprises an amino acid sequence of SEQ ID NO: 12, and
      (iii) CDRH3 comprises an amino acid sequence of SEQ ID NO: 13,
   (b) the light chain variable region comprises CDRL1, CDRL2 and CDRL3,
      (iv) CDRL1 comprises an amino acid sequence of SEQ ID NO: 14,
      (v) CDRL2 comprises an amino acid sequence of SEQ ID NO: 15, and
      (vi) CDRL3 comprises an amino acid sequence of SEQ ID NO: 16.
[29] The composition described in any of [1] to [24], wherein a response in the individual after discontinuation of treatment is sustained by the treatment.
[30] The composition described in any of [1] to [5] and [15] to [29], wherein the individual has hormone receptor-positive HER2-negative breast cancer.
   [30-1] The composition described in any one of [15] to [30], wherein the composition comprises administering anti-PD-Ll antibody at a dose of 840 mg/body once every two weeks.
   [30-1] The composition described in any of [15] to [30], wherein the composition comprises administering paclitaxel each at a dose of 90 mg/m² (body surface area) once a week (28 days as one cycle and administered on days 1, 8 and 15), administering the anti-PD-Ll antibody at a dose of 840 mg/body once every two weeks, and administering the anti-VEGF antibody at a dose of 10 mg/kg (body weight) every two weeks.
   [30-2] The composition described in any of [15] to [30] and [30-1], wherein, by the use of the composition, a median value of progression-free survival period of the individual is elongated as compared with an individual having hormone receptor-positive HER2-negative breast cancer treated with paclitaxel and the anti-VEGF antibody.
   [30-2] The composition described in any of [15] to [30], [30-1] and [30-2], wherein by the use of the composition, a median value of progression-free survival period of the individual is elongated at least about 5 months as compared with an individual having hormone receptor-positive HER2-negative breast cancer treated with paclitaxel and the anti-VEGF antibody.

In some embodiments, by the treatment, a response in the individual after discontinuation of treatment is sustained. In some embodiments, by the treatment, complete remission, partial remission or stabilization of disease in the subject can be given. In some embodiments, the cancer can be in early stage or late stage.

The pharmaceutical composition according to the present disclosure is a pharmaceutical composition for treating estrogen receptor-positive HER2-negative breast cancer in an individual, which contains an anti-PD-Ll antibody, and is a pharmaceutical composition for use in combination with paclitaxel and an anti-VEGF antibody.

The pharmaceutical composition according to the present disclosure is a pharmaceutical composition for treating estrogen receptor-positive HER2-negative breast cancer in an individual, which comprises an anti-VEGF antibody, and is a pharmaceutical composition to be used in combination with paclitaxel and an anti-PD-L1 antibody.

The pharmaceutical composition according to the present disclosure is a pharmaceutical composition for treating estrogen receptor-positive HER2-negative breast cancer in an individual, which comprises paclitaxel, and is a pharmaceutical composition to be used in combination with an anti-PD-Ll antibody and an anti-VEGF antibody.

The pharmaceutical composition according to the present disclosure is a pharmaceutical composition for treating estrogen receptor-positive HER2-negative breast cancer in an individual, and is a pharmaceutical composition comprising paclitaxel, an anti-VEGF antibody and an anti-PD-Ll antibody.

The pharmaceutical composition according to the present disclosure is a pharmaceutical composition for treating estrogen receptor-positive HER2-negative breast cancer in an individual, which is a pharmaceutical composition to be administered in combination with paclitaxel, an anti-PD-Ll antibody and an anti-VEGF antibody.

In any of the above-mentioned pharmaceutical compositions according to the present disclosure, paclitaxel, the anti-PD-Ll antibody and the anti-VEGF antibody may be administered simultaneously or separately and consecutively.

The pharmaceutical composition according to the present disclosure is a pharmaceutical composition for treating estrogen receptor-positive HER2-negative breast cancer, which is a pharmaceutical composition using paclitaxel and an anti-VEGF antibody in combination, wherein paclitaxel, the anti-PD-Ll antibody and the anti-VEGF antibody are administered simultaneously or separately continuously, and which is a pharmaceutical composition comprising the anti-PD-Ll antibody that improves efficacy in a group containing individuals necessary for treating estrogen receptor-positive HER2-negative breast cancer, as compared with the case where the anti-PD-Ll antibody is administered alone, or as compared with the case where paclitaxel is administered alone, or as compared with the case where the anti-VEGF antibody is administered alone, as compared with the case where paclitaxel and the anti-VEGF antibody are administered in combination, as compared with the case where the anti-PD-Ll antibody and the anti-VEGF antibody are administered in combination, or as compared with the case where paclitaxel and anti-PD-L1 antibody are administered in combination.

The pharmaceutical composition according to the present disclosure is a pharmaceutical composition for treating hormone receptor-positive HER2-negative breast cancer, which is a pharmaceutical composition for use in combination with paclitaxel and bevacizumab, in which paclitaxel, atezolizumab and bevacizumab are administered simultaneously or separately continuously, and which is a pharmaceutical composition containing atezolizumab that makes a difference in a reduction in exacerbation risk and or a median value of progression-free survival period in a group containing individuals necessary for treating hormone receptor-positive HER2-negative breast cancer, as compared with the case where paclitaxel and bevacizumab are administered in combination. In a certain embodiment, in the case of combination therapy with paclitaxel, atezolizumab and bevacizumab, exacerbation risk is reduced. In a certain embodiment, in the case of combination therapy with paclitaxel, atezolizumab and bevacizumab, a median value of progression-free survival period is elongated. In a certain embodiment, in the case of combination therapy with paclitaxel, atezolizumab and bevacizumab, exacerbation risk is reduced and a median value of progression-free survival period is elongated.

The pharmaceutical composition according to the present disclosure is a pharmaceutical composition for treating hormone receptor-positive HER2-negative breast cancer, which is a pharmaceutical composition for use in combination with paclitaxel and bevacizumab, in which paclitaxel, atezolizumab and bevacizumab are administered simultaneously or separately continuously, and which is a pharmaceutical composition comprising atezolizumab that makes a difference in a reduction in exacerbation risk and or a median value of progression-free survival period in a group containing individuals necessary for treating hormone receptor-positive HER2-negative breast cancer and who are PD-L1 positive, as compared with the case where paclitaxel and bevacizumab are administered in combination. In a certain embodiment, in a group containing individuals who are PD-L1 positive, exacerbation risk is reduced in the case of combination therapy of paclitaxel, atezolizumab and bevacizumab. In a certain embodiment, in a group containing individuals who are PD-L1 positive, a median value of progression-free survival period is elongated in the case of combination therapy of paclitaxel, atezolizumab and bevacizumab. In a certain embodiment, in a group containing individuals who are PD-L1 positive, exacerbation risk is reduced and a median value of progression-free survival period is elongated in the case of combination therapy of paclitaxel, atezolizumab and bevacizumab.

The pharmaceutical composition according to the present disclosure is a pharmaceutical composition for treating hormone receptor-positive HER2-negative breast cancer, which is a pharmaceutical composition for use in combination with paclitaxel and bevacizumab, and is a pharmaceutical composition containing atezolizumab that makes a difference in a reduction in exacerbation risk and or a median value of progression-free survival period in a group containing individuals who are recurrent breast cancer at the start of this treatment and in a group comprising individuals who are stage IV in which paclitaxel, atezolizumab and bevacizumab are administered simultaneously or separately continuously, necessary for treating hormone receptor-positive HER2-negative breast cancer, as compared with the case where paclitaxel and bevacizumab are administered in combination. In a certain embodiment, in a group containing individuals who are recurrent breast cancer at the start of this treatment, exacerbation risk is reduced in the case of combination therapy of paclitaxel, atezolizumab and bevacizumab. In a certain embodiment, in a group containing individuals who are recurrent breast cancer at the start of this treatment, a median value of progression-free survival period is elongated in the case of combination therapy of paclitaxel, atezolizumab and bevacizumab. In a certain embodiment, in a group containing individuals who are recurrent breast cancer at the start of this treatment, exacerbation risk is reduced and a median value of progression-free survival period is elongated in the case of combination therapy of paclitaxel, atezolizumab and bevacizumab.

The pharmaceutical composition according to the present disclosure is a pharmaceutical composition for treating hormone receptor-positive HER2-negative breast cancer, which is a pharmaceutical composition for use in combination with paclitaxel and bevacizumab, and is a pharmaceutical composition comprising atezolizumab that makes a difference in a reduction in exacerbation risk and or a median value of progression-free survival period in a group in which paclitaxel, atezolizumab and bevacizumab are administered simultaneously or separately continuously, necessary for treating hormone receptor-positive HER2-negative breast cancer and containing individuals having no liver metastases at the start of this treatment, as compared with the case where paclitaxel and bevacizumab are administered in combination, and as compared with the case where paclitaxel and bevacizumab are administered in combination in a group containing individuals having liver metastases. In a certain embodiment, in a group containing individuals having liver metastases at the start of this treatment, exacerbation risk is reduced in the case of combination therapy of paclitaxel, atezolizumab and bevacizumab. In a certain embodiment, in a group containing individuals having liver metastases at the start of this treatment, a median value of progression-free survival period is elongated in the case of combination therapy of paclitaxel, atezolizumab and bevacizumab. In a certain embodiment, in a group containing individuals having liver metastases at the start of this treatment, exacerbation risk is reduced and a median value of progression-free survival period is elongated in the case of combination therapy of paclitaxel, atezolizumab and bevacizumab.

Use according to the present disclosure is a use of paclitaxel, an anti-PD-Ll antibody and an anti-VEGF antibody for manufacturing the pharmaceutical composition of the present disclosure, or the kit.

The combination of the present disclosure is a combination of paclitaxel, an anti-VEGF antibody and an anti-PD-Ll antibody, which is a combination for use in treating estrogen receptor-positive HER2-negative breast cancer.

### EFFECTS OF THE INVENTION

The present disclosure is useful for treatment of estrogen receptor-positive HER2-negative breast cancer or hormone receptor-positive HER2-negative breast cancer.

### BRIEF DESCRIPTION OF THE DRAWINGS

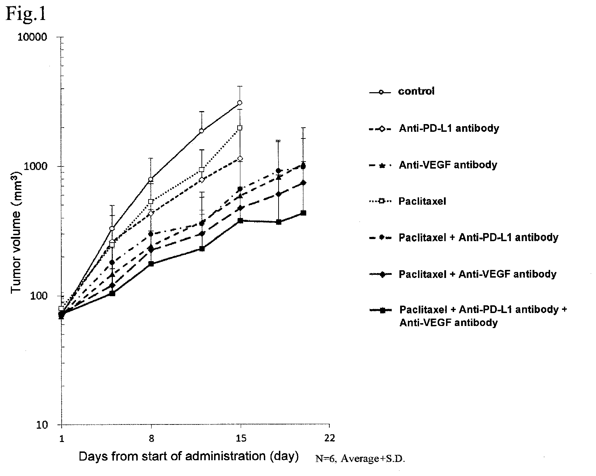

Fig. 1 is a drawing showing a tumor volume change of mouse breast cancer cell strain FM3A in each mouse to which any of solvent control (■), single agent administration (5 mg/kg of 10F.9G2 (anti-PD-L1 antibody according to both routes of PD-L1 and PD-1, and PD-L1 and B7-1) twice per week; (▲), 10 mg/kg of B20-4.1.1 (anti-VEGF antibody) once per week; (◇), or 100 mg/kg of paclitaxel once per week; (•), 2 agents combination administration (paclitaxel + 10F.9G2; ◇◇, paclitaxel + B20-4.1.1; □) or 3 agents combination administration (paclitaxel + 10F. 9G2 + B20-4.1.1; o) is administered. Six mice per each group were treated. Average of tumor volume in each group and SD bar were plotted. The vertical axis is the tumor volume (mm³), and the horizontal axis is a number of days.

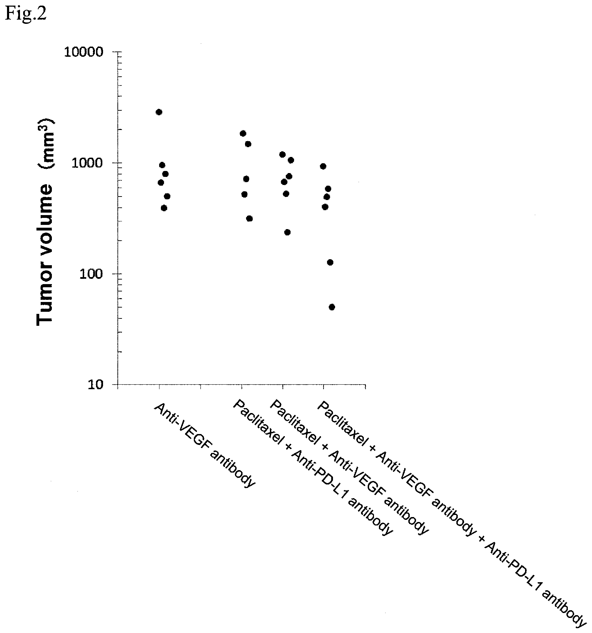

Fig. 2 is a drawing plotting a tumor volume (mm³) on day 20 after the start of administration of a solvent control group and each administered group in which either of 5 mg/kg of 10F.9G2, 10 mg/kg of B20-4.1.1, 100 mg/kg of paclitaxel or a combination thereof is administered. Six mice per each group were treated. The vertical axis is a number of CD8 positive T cells per 1 ml. The horizontal axis is anti-VEGF antibody, paclitaxel + anti-PD-Ll antibody, paclitaxel + anti-VEGF antibody, and paclitaxel + anti-PD-L1 antibody + anti-VEGF antibody in combination from left.

### EMBODIMENTS TO CARRY OUT THE INVENTION

### I. Definition

Before explaining the present disclosure in detail, it should be understood that the present disclosure is not limited to particular compositions or biological systems, and it is to be understood that these may vary as a matter of course. In addition, it is also to be understood that the technical terms used in the present specification are intended for the purpose of describing specific embodiments only and is not intended to be limiting.

### I. General techniques

The techniques and procedures described or referred to herein are generally well understood and widely employed by those skilled in the art using conventional methodologies. Such conventional methodologies are, for example, the widely used methodologies described below: Sambrook et al., Molecular Cloning: A Laboratory Manual 3d edition (2001) Cold Spring Harbor Laboratory Press, Cold Spring Harbor, N. Y.; Current Protocols in Molecular Biology (F. M. Ausubel, et al. eds., (2003)); the series Methods in Enzymology (Academic Press, Inc.): PCR 2: A Practical Approach (M. J. MacPherson, B. D. Hames and G. R. Taylor eds. (1995)), Harlow and Lane, eds. (1988) Antibodies, A Laboratory Manual, and Animal Cell Culture (R. I. Freshney, ed. (1987)); Oligonucleotide Synthesis (M. J. Gait, ed., 1984); Methods in Molecular Biology, Humana Press; Cell Biology: A Laboratory Notebook (J. E. Cellis, ed., 1998) Academic Press; Animal Cell Culture (R. I. Freshney), ed., 1987); Introduction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds., 1993-8) J. Wiley and Sons; Handbook of Experimental Immunology (D. M. Weir and C. C. Blackwell, eds.); Gene Transfer Vectors for Mammalian Cells (J. M. Miller and M. P. Calos, eds., 1987); PCR: The Polymerase Chain Reaction, (Mullis et al., eds., 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V. T. DeVita et al., eds., J. B. Lippincott Company, 1993), New guidelines for judging the therapeutic effect of solid cancer (RECIST guideline) Revised version 1.1-Japanese translation JCOG version-: Revised RECIST guideline (version 1.1) (http://www.jcog.jp/doctor/tool/RECISTv11J_20100810.pdf), EUROPEAN JOURNAL OF CANCER 45 (2009) 228-247.

The singular forms of "a", "an" and "the" used in the present specification and in the accompanying drawings also include multiple designators, unless the content clearly indicates a contrary meaning. Accordingly, for example, a reference to "a molecule" optionally includes a combination of two or more such molecules, etc.

The term "about (approximately)" as used in the present specification means the normal range of error for each value immediately known to those skilled in the art. References to a value or parameter with "about (approximately)" in the present specification include (and explain) embodiments directed to the value or parameter itself. That is, about 5 months may be 5.4 months, 5.3 months, 5.2 months, 5.1 months, 5.0 months, 4.9 months, 4.8 months, 4.7 months, 4.6 months, 4.5 months (rounded off to 5 months), about 4 months may be 4.4 months, 4.3 months, 4.2 months, 4.1 months, 4.0 months, 3.9 months, 3.8 months, 3.7 months, 3.6 months, 3.5 months (rounded off to 4 months), and about 3 months may be 3.4 months, 3.3 months, 3.2 months, 3.1 months, 3.0 months, 2.9 months, 2.8 months, 2.7 months, 2.6 months, 2.5 months (rounded off to 3 months).

The aspects and embodiments of the present disclosure described in the present specification are to be understood to include "containing," "comprising," and "consisting essentially of' these aspects and embodiments.

The terms "completely effective," "complete remission" or "CR (complete response)" used in the present specification mean disappearance of all target lesions.

The terms "partially effective," "partial remission" or "PR (partial response)" used in the present specification mean a reduction of at least 30% of the baseline sum of longest diameter (SLD) of the target lesion, based on the SLD.

The terms "stable," "disease stable" or "SD (stable disease)" used in the present specification mean that there is neither sufficient shrinkage of the target lesion to make it suitable for PR nor sufficient enlargement to make it suitable for PD, based on the minimum SLD since treatment began.

The terms "progression", "disease progression" or "PD (progressive disease)" used in the present specification mean an increase of at least 20% of the SLD of the target lesion or the presence of one or more new lesions, based on the smallest SLD recorded since the start of treatment.

The terms "sustained remission" mean a sustained effect on the reduction of tumor growth after discontinuation of treatment. For example, the tumor size may remain the same or smaller compared to its size at the beginning of the administration phase. In a part of the embodiment, sustained remission has at least as long as the treatment duration, for example, 1. 5 times, 2. 0 times, 2. 5 times or 3. 0 times the treatment duration.

The terms "survival period" used in the present specification refers to a patient is alive, and includes overall survival (OS) and progression-free survival period (PFS).

The terms "overall survival" or "OS" used in the present specification refers to the fact that a patient is still alive over a predetermined period of time such as 1 year, 5 years, 12 years, 10 years, 15 years, etc., from the time of diagnosis or treatment. For the clinical trials described in the examples, overall survival (OS) is defined as the time from the date of randomization of the patient population to the date of death from any cause.

The terms "progression-free survival period" or "PFS" used in the present specification refers to the fact that the patient is still alive without the cancer progressing or worsening. For the clinical trials described in the examples, progression-free survival period (PFS) is defined as the time from randomization of the test population from any of the first occurrence of death by any of the cause of the first described progressive disease or unmanageable toxicity, or any of the cause. Disease progression can be determined by any clinically acceptable method, for example, progressive disease on radiographic examination as determined by the Solid Tumor Evaluation Criteria (RECIST: EUROPEAN JOURNAL OF CANCER 45 (2009) 228-247), cancerous meningitis diagnosed by cellular evaluation of cerebrospinal fluid, and/or medical photography to monitor chest wall recurrence of subcutaneous lesions.

The terms "survival period is elongated" used in the present specification mean to increase overall survival or progression-free survival period in patients treated in accordance with the present disclosure as compared to untreated patients, and/or as compared to patients treated with one or more approved antitumor agents but not treated in accordance with the present disclosure. In a specific example, "survival period is elongated" means elongating progression-free survival period (PFS) and/or overall survival (OS) of cancer patients receiving the combination therapy (treatment with the combination of atezolizumab, bevacizumab and paclitaxel) of the present disclosure as compared to patients treated bevacizumab and paclitaxel alone. In another specific example, "survival period is elongated" means elongating progression-free survival period (PFS) and/or overall survival (OS) of cancer patients receiving the combination therapy (treatment with the combination of atezolizumab, bevacizumab and paclitaxel) of the present disclosure as compared to patients treated bevacizumab and paclitaxel alone.

The term "treatment" used in the present specification means a clinical intervention intended to alter the natural course of the individual or cells being treated during the course of clinical pathology. In desired effects of treatment, a reduction in the rate of disease progression, improvement or alleviation of the disease state, and remission or improved prognosis are contained. For example, "treatment" is said to be succeeded when one or more symptoms associated with cancer of individuals including reduction (or destruction) of growth of cancer cells, reduction of symptoms as a result of the disease, improvement in the quality of life of the person suffered from the disease, and reduction in the dose of other drugs required to treat the disease and/or elongation of life of individual (but not limited to these).

The terms "delaying progression" of the disease used in the present specification mean to prolong, hinder, decelerate, delay, stabilize and/or postpone the occurrence of a disease (for example, cancer). The temporal length of this delay can vary depending on the history of the disease and/or the history of the individual being treated. As will be obvious to those skilled in the art, sufficient or significant delay encompasses prevention in the sense that the individual virtually never develops the disease. For example, it may delay terminal cancer, such as the development of metastases.

An "effective amount" is the minimum amount necessary to achieve at least measurable improvement or prevention of a specific disorder. In the present specification, the effective amount may vary depending on factors such as the patient's medical condition, age, sex, weight, and the ability of the active substance to elicit the desired response in the individual. In addition, the effective amount is also an amount at which the therapeutically beneficial effect outweighs any toxic or detrimental effects of the treatment. In the case of prophylactic use, in the beneficial or desired results, it contains the results of eliminating or reducing the risk of, reducing the severity of, or delaying the onset of the disease, including biochemical, histological and/or behavioral symptoms of the disease, its complications, and intermediate pathological phenotypes that emerge during development of the disease. In the case of therapeutic use, in the beneficial or desired results, it contains the clinical results of reducing one or more symptoms as a result of the disease, improving the quality of life of the person suffered from the disease, reducing the dose of another drug needed to treat the disease and enhancing the effect of another drug, such as by targeting, slowing the progression of the disease, and/or prolonging life. In the case of a cancer or tumor, an effective amount of the medicament may have a possibility to have effects in reducing the number of cancer cells; reducing the size of the tumor; inhibiting invasion of the cancer cells into surrounding organs (that is, slowing to some extent, desirably stopping); inhibiting metastasis of tumor (that is, slowing to some extent, desirably stopping); inhibiting growth of tumor to some extent; and/or relieving one or more symptoms associated with the disorder to some extent. The effective amount may be administered in one or more times of administration. In the purpose of the present disclosure, an effective amount of a medicament, compound or pharmaceutical composition is an amount sufficient to achieve, directly or indirectly, prophylactic or therapeutic treatment. As can be understood from a clinical perspective, an effective amount of a medicament, compound or pharmaceutical composition may or may not be achieved in conjunction with another medicament, compound or pharmaceutical composition. Thus, the "effective amount" may be considered in connection with the administration of one or more therapeutic agents, and when a desired result is capable of achieving or can be achieved in combination with one or more other medicaments, a single medicament can be deemed to be given in an effective amount.

The terms "in combination with" used in the present specification means administration of one therapeutic manner to which another therapeutic manner is added. Accordingly, "in combination with" means administration of the other therapeutic manner before, during, or after administration of one therapeutic manner to an individual.

The term "tumor" used in the present specification means all neoplastic cell growth and proliferation, and all precancerous and cancerous cells and tissues whether malignant or benign. The terms "cancer," "cancerous," "cell proliferative disorder," "proliferative disorder," and "tumor," as used herein, are not mutually exclusive as referred to in the present specification.

The terms "cancer" and "cancerous" mean or describe physiological conditions in mammals that are typically characterized by uncontrolled cell growth. Examples of cancer include breast cancer (for example, estrogen receptor positive (ER+) and/or progesterone receptor positive (PR+) and HER2 negative (HER2-) breast cancer, but are not limited thereto. In a certain specific embodiment, cancers sensitive to the treatment with the methods and compositions of the present disclosure can be invasive cancers among breast cancers (for example, estrogen receptor positive and/or progesterone receptor positive and HER2-negative breast cancer. In a certain specific embodiment, cancers sensitive to the treatment with the methods and compositions of the present disclosure can be invasive ductal carcinomas and special types of invasive carcinomas, and the special types include, depending on the histological type, invasive lobular carcinoma, tubular carcinoma, cribriform carcinoma, mucinous carcinoma, medullary carcinoma, apocrine carcinoma, metaplastic carcinoma, invasive micropapillary carcinoma, secretory carcinoma, adenoid cystic carcinoma, etc.

With respect to methods for testing for positive and negative estrogen and progesterone receptors and HER2, it has been demonstrated that ER, PgR and HER2 detected by immunohistochemical methods in formalin-fixed paraffin-embedded tissue can be quantified by computerized image analysis (for example, Journal of Surgical Oncology 1996, Volume 61, Issue 3 p. 177-184.).

Paclitaxel, for example, can be used that is marketed under the trade name Taxol, or that contains paclitaxel in its trade name, or that has paclitaxel as the generic name of the active ingredient.

The anti-PD-L1 antibody can be obtained as a polyclonal antibody or monoclonal antibody using known means. The origin of the antibody is not particularly limited, and it is preferably of mammalian origin, and more preferably of human origin antibody. As the mammalian-derived monoclonal antibody, there are those produced in hybridoma, and those produced in hosts transformed with expression vectors containing antibody genes by genetic engineering means.

An anti-PD-Ll antibody is an antibody that binds to PD-L1. In a certain embodiment, the anti-PD-Ll antibody inhibits binding of PD-L1 to both PD-1 and B7-1. In some embodiments, the antibody is selected from the group consisting of MPDL3280A (atezolizumab), YW243.55.S70, PRO304397, PRO314483 and 10F.9G2. In some embodiments, the antibody is atezolizumab.

Inhibiting function of PD-L1 refers to releasing the suppression of activation of T cells caused by the result of binding of PD-1 or B7.1 and PD-L1, and the activity of PD-L1 is reduced by at least 5%, 10%, 20%, 30%, 40%, 50%, 60%, 70%, 80%, 90% or 100% compared to activity in control. The activity of PD-L1 is determined by an optional standard method in the field of the art (including those described in the present specification).

The anti-PD-Ll antibody useful for the present disclosure include a composition containing such an antibody, such as those described in WO 2010/077634A1, which can be used in combination with paclitaxel to treat cancer, with or without a VEGF antagonist.

In one embodiment, the anti-PD-Ll antibody is atezolizumab (CAS registry number: 1422185-06-5), atezolizumab (Genentech), and an anti-PD-L1 antibody also known as MPDL3280A.
[A1] Atezolizumab is an anti-PD-Ll antibody which comprises a heavy chain variable region polypeptide comprising HVR-H1, HVR-H2 and HVR-H3 sequences, wherein:
   (a) HVR-H1 sequence is GFTFSDSWIH (SEQ ID NO: 1),
   (b) HVR-H2 sequence is AWISPYGGSTYYADSVKG (SEQ ID NO: 2),
   (c) HVR-H3 sequence is RHWPGGFDY (SEQ ID NO: 3), and
   a light chain variable region polypeptide comprising HVR-L1, HVR-L2 and HVR-L3, wherein:
   (a) HVR-L1 sequence: RASQDVSTAVA (SEQ ID NO: 4);
   (b) HVR-L2 sequence: SASFLYS (SEQ ID NO: 5); and
   (c) HVR-L3 sequence: QQYLYHPAT (SEQ ID NO: 6).
[A2] Atezolizumab comprises heavy chain and light chain variable region sequences, wherein:
   (a) the heavy chain variable region sequence is: 


 (SEQ ID NO: 7), and
   (b) the light chain variable region sequence is: 
DIQMTQSPSSLSASVGDRVTITCRASQDVSTAVAWYQQKPGKAPKLLIYSASF
LYSGVPSRFSGSGSGTDFTLTISSLQPEDFATYYCQQYLYHPATFGQGTKVEIKR
(SEQ ID NO: 8).
[A3] Atezolizumab comprises heavy chain and light chain sequences, wherein:
   (a) the heavy chain sequence is: 
EVQLVESGGGLVQPGGSLRLSCAASGFTFSDSWIHWVRQAPGKGLEWVAWISP
YGGSTYYADSVKGRFTISADTSKNTAYLQMNSLRAEDTAVYYCARRHWPGGF
DYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYASTYRVVSVLTVLHQDWLNGK
EYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFS
CSVMHEALHNHYTQKSLSLSPG (SEQ ID NO: 9), and
   (b) the light chain sequence is:

DIQMTQSPSSLSASVGDRVTITCRASQDVSTAVAWYQQKPGKAPKLLIYSASFL
YSGVPSRFSGSGSGTDFTLTISSLQPEDFATYYCQQYLYHPATFGQGTKVEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
(SEQ ID NO: 10).

### VEGF antagonist

The present disclosure provides a method of treating cancer or delaying progression of cancer in an individual, which method includes administering an effective amount of a PD-1 pathway antagonist and a VEGF antagonist together with paclitaxel. Any known VEGF antagonist is intended.

The term "VEGF" as used herein means 165 amino acid human vascular endothelial growth factor and related 121, 189, and 206 amino acid human vascular endothelial growth factors as described by Leung et al., Science, 246: 1306 (1989), and Houck et al., Mol. Endocrin., 5: 1806 (1991), together with their naturally occurring allelonorphs and processed forms. The term "VEGF" also means VEGF from non-human species such as mouse, rat or primate. VEGF from a specific species is often indicated by terms such as hVEGF for human VEGF, mVEGF for mouseVEGF, etc. The term "VEGF" also used to mean truncated forms of the polypeptide comprising amino acids 8 to 109 or 1 to 109 of the 165 amino acid human vascular endothelial growth factor. A designation of optional such form of VEGF may be made in this application, for example, "VEGF(8-109)," "VEGF(1-109)" or "VEGF₁₆₅". Amino acid positions in the "truncated" naturally occurring VEGF are numbered as shown in the naturally occurring VEGF sequence. For example, amino acid position 17 (methionine) of truncated naturally occurring VEGF is also position 17 (methionine) of naturally occurring VEGF. Truncated naturally occurring VEGF has binding affinities to the KDR and Flt-1 receptors comparable to naturally occurring VEGF. According to a preferred embodiment, the VEGF is human VEGF .

An "anti-VEGF antibody" is an antibody that binds VEGF with sufficient affinity and specificity. The selected antibody generally has a binding affinity for VEGF, for example, the antibody is capable of binding to hVEGF with a Kd value of 100 nM to 1 pM. Antibody affinities can be determined, for example, by surface plasmon resonance-based assays (such as the BIAcore assay described in PCT Application WO 2005/012359, etc.); enzyme-linked immunosorbent assay (ELISA); and competition experiments (for example, RIA). In a specific embodiment, the anti-VEGF antibody of the present disclosure may be useful as a medicament in targeting and interfering with diseases and conditions involving VEGF activity. Also, the antibody may also be subject to other bioactivity assays, for example, to evaluate the efficacy as the therapeutic method. Such assays are known in this field of the art, and determined depending on intended use of the target antigen and antibody. As an example, there may be mentioned HUVEC inhibition assays; tumor cell growth inhibition assays (for example, described in WO 89/06692); antibody-dependent cellular cytotoxicity (ADCC) and complement-mediated cytotoxicity (CDC) assays (US Patent No. 5,500,362); and agonist activity or hematopoietic assays (see WO 95/27062). The anti-VEGF antibody will usually not bind to other VEGF homologs such as VEGF-B, or VEGF-C, etc., nor to other growth factors such as P1GF, PDGF or bFGF, etc.

In a specific embodiment of the present disclosure, the anti-VEGF antibody includes a monoclonal anti-VEGF antibody A4.6.1 produced by hybridoma ATCC HB 10709; and a monoclonal antibody that bind to the same epitope as recombinant humanized anti-VEGF monoclonal antibody produced in accordance with Presta et al. (1997) Cancer Res. 57: 4593-4599, but not limited thereto. In one embodiment, the anti-VEGF antibody is "bevacizumab (BV)" also known as "rhuMAb VEGF" or "AVASTIN (Registered Trademark)". This includes a mutated human IgG1 framework region and antigen-binding complementarity-determining region derived from the mouse anti-hVEGF monoclonal antibody A.4.6.1 that blocks binding of human VEGF to its receptor. Approximately 93% of the amino acid sequence of bevacizumab, including most of the framework region, is derived from human IgG1, and approximately 7% of the sequence is derived from mouse antibody A4.6.1.

Bevacizumab and other humanized anti-VEGF antibodies are further described in U.S. Patent No. 6,884,879, issued February 26, 2005. As further antibodies, G6 or B20 series antibodies (for example, G6-31, B20-4.1) as described in WO 2005/012359, WO 2005/044853 and US Patent Application No. 60/991,302 are included, and it is clearly described that the above-mentioned Patent Documents are expressly incorporated in the present specification by reference. With regard to the further antibodies, see US Patent No. 7,060,269, Ditto 6,582,959, Ditto 6,703,020; Ditto 6,054,297; WO 98/45332; Ditto 96/30046; Ditto 94/10202; EP0666868B1; U.S. Patent Application Publication No. 2006009360, Ditto 20050186208, Ditto 20030206899, Ditto 20030190317, Ditto 20030203409, and Ditto 20050112126; and Popkov et al., Journal of Immunological Methods 288: 149-164 (2004). Other antibodies include those that bind to functional epitopes on human VEGF comprising residues F17, M18, D19, Y21, Y25, Q89,191, K101, E103 and C104, or, alternatively, residues F17, Y21, Q22, Y25, D63, I83 and Q89.

In one embodiment of the present disclosure, the anti-VEGF antibody comprises a heavy chain variable region comprising the following amino acid sequence: EVQLVESGGGLVQPGGSLRLSCAASGYTFTNYGMNWVRQAPGKGLEWVGWI NTYTGEPTYAADFKRRFTFSLDTSKSTAYLQMNSLRAEDTAVYYCAKYPHYYG SSHWYFDVWGQGTLVTVSS (SEQ ID NO: 17),
and a light chain variable region comprising the following amino acid sequence:
DIQMTQSPSSLSASVGDRVTITCSASQDISNYLNWYQQKPGKAPKVLIYFTSSLH SGVPSRFSGSGSGTDFTLTISSLQPEDFATYYCQQYSTVPWTFGQGTKVEIKR (SEQ ID NO: 18).

In some embodiments, the anti-VEGF antibody comprises
CDRH1 comprising the following amino acid sequence: GYTFTNYGMN (SEQ ID NO: 11),
CDRH2 comprising the following amino acid sequence: WINTYTGEPTYAADFKR (SEQ ID NO: 12),
CDRH3 comprising the following amino acid sequence: YPHYYGSSHWYFDV (SEQ ID NO: 13),
CDRL1 comprising the following amino acid sequence: SASQDISNYLN (SEQ ID NO: 14),
CDRL2 comprising the following amino acid sequence: FTSSLHS (SEQ ID NO: 15), and
CDRL3 comprising the following amino acid sequence: QQYSTVPWT (SEQ ID NO: 16).

The "B20 series antibody" according to the present disclosure is an anti-VEGF antibody derived from the sequence of a B20 antibody or a B20-derived antibody described in any one of Figs. 27 to 29 of WO 2005/012359, and the full disclosure of which is incorporated in the present specification by reference. In addition, WO 2005/044853 and US Patent Application No. 60/991302, the contents of which are incorporated in the present specification by reference, are also referred to. In one embodiment, the antibody of B20 series binds to functional epitopes on human VEGF containing residues F17, M18, D19, Y21, Y25, Q89,191, K101, E103 and C104.

A "functional epitope" according to the present disclosure refers to an amino acid residue of an antigen that energetically contributes to binding of an antibody. Mutation of any one of the residues of the antigen that energetically contributing (for example, mutation of wild-type VEGF by mutation of alanine or homologue) would disrupt the binding of the antibody, and in which case, the ratio of relative affinity of the antibody (IC50 mutant VEGF/IC50 wild-type VEGF) exceeds 5 (see Example 2 of WO 2005/012359)). In one embodiment, the ratio of relative affinity is determined by solution-bound phage expressing ELISA. Briefly explaining, 96-well Maxisorp immunoplates (NUNC) are coated overnight at 4°C with Fab forms of the antibody under test at a concentration of 2 ug/ml in PBS and blocked for 2 hours at room temperature using PBS, 0.5% of BSA and 0.05% of Tween20 (PBT). A material in which phage expressing hVEGF alanine point mutant (form of residues 8 to 109) or wild-type hVEGF (8 to 109) is serially diluted is firstly incubated in PBT on a Fab-coated plate at room temperature for 15 minutes, and the plate is washed with PBS and 0.05% Tween 20 (PBST). The phage bound by anti-M13 monoclonal antibody horseradish peroxidase (Amersham Pharmacia) conjugate diluted 1 : 5,000 in PBT is detected, grown by 3,3',5,5'-tetramethylbenzidin (TBM, Kirkegaard & Perry Labs, Gaithersburg, MD) substrate for about 5 minutes, quenched using 1.0 M H3PO4, and read by spectrophotometry at 450 nm. The ratio of IC50 value (IC50, ala/IC50, wt) represents the multiple of the decrease in bonding affinity (relative bonding affinity).

### IV. Kit

In another embodiment, it is provided a kit containing a PD-L1 system binding antagonist and/or a VEGF antagonist for the purpose of treating cancer or delaying progression of cancer in an individual, or enhancing immune function of an individual. In some embodiments, the kit contains a PD-1 system binding antagonist, and a package insert containing instructions for using the PD-1 system binding antagonist in combination with paclitaxel with or without a VEGF antagonist in order for treating cancer or delaying progression of cancer in an individual, or enhancing immune function of an individual. In some embodiments, the kit contains paclitaxel with or without a VEGF antagonist, and a package insert containing instructions for using the PD-1 system binding antagonist in combination with paclitaxel with or without a VEGF antagonist in order for treating cancer or delaying progression of cancer in an individual, or enhancing immune function of an individual. In some embodiments, the kit contains a PD-1 system binding antagonist and paclitaxel with or without a VEGF antagonist, and a package insert containing instructions (attached document) for using a PD-1 system binding antagonist and paclitaxel with or without a VEGF antagonist in order for treating cancer or delaying progression of cancer in an individual, or enhancing immune function of an individual. Any of the PD-1 system binding antagonist and/or the VEGF antagonist described in the present specification can be included in the kit.

As the anti-VEGF antibody, for example, bevacizumab, ramucirumab, and aflibercept beta can be used. In some embodiments, the anti-VEGF antibody binds to the same epitope as monoclonal anti-VEGF antibody A4.6.1 produced by hybridoma ATCC HB 10709. The anti-VEGF antibody can be made a humanized antibody or a human antibody. In some embodiments, the anti-VEGF antibody is bevacizumab. In some embodiments, anti-VEGF antibody comprises a heavy chain variable region comprising an amino acid sequence: 


 (SEQ ID NO: 17), and
 a light chain variable region comprising an amino acid sequence:

DIQMTQSPSSLSASVGDRVTITCSASQDISNYLNWYQQKPGKAPKVLIYFTSSLH

SGVPSRFSGSGSGTDFTLTISSLQPEDFATYYCQQYSTVPWTFGQGTKVEIKR
(SEQ ID NO: 18).

The term "antibody" includes a monoclonal antibody (including full-length antibodies having immunoglobulin Fc regions), an antibody composition having polyepitope specificity, a multi-specific antibody (for example, a bispecific antibody, diabody and single chain molecule), and an antibody fragment (for example, Fab, F(ab')₂ and Fv). The term "immunoglobulin" (Ig) is used in the present specification as the same meaning of "antibody".

The basic four-chain antibody unit is a heterotetrameric glycoprotein composed of two identical light (L) chains and two identical heavy (H) chains. In the case of IgG, four-chain unit is generally about 150,000 daltons. Each L chain is linked to an H chain by one covalent disulfide bond, while the two H chains are linked to each other by one or more disulfide bonds depending on the H chain isotype. Also, each H and L chain has regularly spaced intrachain disulfide bridges. Each H chain has a variable domain (V_{H}) at the N-terminus, followed by three constant domains (C_{H}) for the α and γ chains, respectively, and four C_{H} domains for the µ and ε isotypes. Each L chain has a variable domain (V_{L}) at the N-terminus, followed by a constant domain (C_{L}) at its other end. The V_{L} is aligned with the V_{H} and the C_{L} is aligned with the first constant domain (C_{H}1 ) of the heavy chain. Specific amino acid residues are thought to form the interface between the variable domains of the light chain and heavy chain. V_{H} and V_{L} become a pair to form a single antigen binding site. L chain derived from optional vertebrate species can be assigned to one of two distinct types, called kappa and lambda, based on the amino acid sequence of their constant domains. Based on the amino acid sequence of the constant domain (CH) of these heavy chains, immunoglobulins can be assigned to various classes or isotypes. There are five major classes of immunoglobulins, i.e.: IgA, IgD, IgE, IgG and IgM having heavy chains designated α, δ, ε, γ and µ, respectively. The γ and α classes are further divided into subclasses based on relatively minor differences in CH sequence and function and, for example, in humans, it expresses the following subclasses: IgG1, IgG2A, IgG2B, IgG3, IgG4, IgA1 and IgA2.

The "variable region" or "variable domain" of the antibody refers to the N-terminal domain of the heavy chain or light chain of an antibody. The variable domains of the heavy chain and light chain are also referred to as "VH" and "VL". These domains are generally the most variable parts of the antibody (as compared with other antibodies of the same class) and contain the antigen binding sites.

The term "variable" means that certain segments of the variable domains differ extensively in sequence among antibodies. The V domain mediates antigen binding and defines the specificity of a specific antibody for its specific antigen. However, the variability is not evenly distributed across the entire span of the variable domain. Instead, it concentrates on three segments called hypervariable region (HVR) of both the light chain and heavy chain variable domains. The more highly conserved portions of variable domains are called the framework regions (FR). The variable domains of naturally occurring heavy chain and light chain each contain four FR regions, and mainly adopts β-sheet arrangement joined by three HVRs that form loop bindings that connect (and sometimes form part thereof) the β-sheet structure. The HVRs of each chain are held together in close proximity by the FR region and, together with HVRs derived from other chains, contribute to formation of the antigen binding site of the antibody (see Kabat et al., Sequences of Immunological Interest, Fifth Edition, National Institute of Health, Bethesda, MD (1991)). Constant domains are not directly related to antibody binding to antigens, but indicate the involvement of the antibody in various effector functions, for example, antibody-dependent cytotoxicity.

The terms "monoclonal antibody" used in the present specification refer to an antibody obtained from a substantially homogeneous population of antibodies, that is, individual antibodies, including this population, are identical except for possible naturally occurring mutations and/or post-translational modifications (for example, isomerization, amidation) that may be present in trace amounts. The monoclonal antibody is highly specific and a material to a single antigenic site. In contrast to polyclonal antibody preparations, which typically contain different antibodies to different determinants (epitopes), each monoclonal antibody is directed against a single determinant on the antigen. In addition to these specificity, monoclonal antibodies are advantageous in that they are synthesized by hybridoma culture in which no other immunoglobulins are mixed. The modifier "monoclonal" indicates the characteristic of antibodies obtained from a substantially homogeneous population of antibodies and should not be interpreted as requiring the production of antibodies by any particular method. For example, monoclonal antibodies used in accordance with the present disclosure can be produced by various techniques, which techniques include, for example, hybridoma methods (for example, Kohler and Milstein., Nature, 256: 495-97 (1975); Hongoet al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N. Y., 1981)), recombinant DNA methods (for example, see U.S. Patent No. 4,816,567), phage display technology (for example, Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132 (2004)), as well as techniques for generating human or human-like antibodies in animals having genes encoding part or all of a human immunoglobulin locus or a human immunoglobulin sequence (for example, see WO 98/24893; WO 96/34096; WO 96/33735; WO 91/10741; Jakobovits et al., Proc. Natl. Acad. Sci. USA90: 2551 (1993); Jakobovits et al., Nature 362: 255-258 (1993); Bruggemann et al., Year in Immunol. 7: 33 (1993); U.S. Patent No. 5,545,807; Ditto 5,545,806; Ditto 5,569,825; Ditto 5,625,126; Ditto 5,633,425; Ditto 5,661,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg and Huszar, Intern. Rev. Immunol. 13: 65-93 (1995)).

The monoclonal antibody of the present disclosure specifically contains a "chimeric" antibody (immunoglobulin), wherein portions of a heavy chain and/or light chain are identical or homologous to corresponding sequences in antibodies derived from a specific species or belonging to a specific antibody class or subclass, but the remainder of its (their) chains are identical or homologous to corresponding sequences in antibodies derived from another species or belonging to another antibody class or subclass, and fragments of such antibodies (provided that it is required that they exhibit the desired biological activity) (U.S. Patent No. 4,816,567; Morrison et al., Proc. Natl. Acad. Sci. USA, 81: 6851-6855 (1984)). In the chimeric antibody which is an object in the present specification, a PRIMATIZED (Registered Trademark) antibody is contained, and the antigen binding region of this antibody is, for example, derived from an antibody generated by immunizing macaque monkeys using the target antigen. The "humanized antibody" used in the present specification is used as a subset of "chimeric antibody".

A "humanized" form of a non-human (for example, mouse) antibody is a chimeric antibody comprising the minimal sequence derived from a non-human immunoglobulin. In one embodiment, a humanized antibody is a human immunoglobulin (recipient antibody) in which residues of the recipient's HVR (defined below) are replaced by residues of the HVR of a non-human species (donor antibody) such as mouse, rat, rabbit or non-human primate with desired specificity, affinities and/or ability. In some examples, the framework ("FR") residue of the human immunoglobulin are replaced by corresponding non-human residue. Further, a humanized antibody can contain residues not found in the recipient antibody or donor antibody. These modifications may be made to further refine characteristics of the antibody such as bonding affinity. In general, the humanized antibody comprises at least one, typically nearly all of two variable domains, and in such variable domains, all or nearly all of the high frequency variable loops correspond to the same loops of non-human immunoglobulin sequences and all or nearly all of the FR regions correspond to the same regions of human immunoglobulin sequences, and the FR regions may have substitutions of one or a plural of individual FR residues that can improve antibody characteristics such as bonding affinity, isomerization, immunogenicity, etc. A number of such amino acid substitutions in RF is typically 6 or less in H chains and 3 or less in L chains. The humanized antibody will also contain, with optional selection, at least a portion (Fc) of the constant region of an immunoglobulin, typically that of a human immunoglobulin. For further details, see, for example, Jones et al., Nature 321: 522-525 (1986); Riechmann et al., Nature 332: 323-329 (1988); and Presta, Curr. Op. Struct. Biol. 2: 593-596 (1992). In addition, also see, for example, Vaswani and Hamilton, Ann. Allergy, Asthma & Immunol. 1: 105-115 (1998); Harris, Biochem. Soc. Transactions 23: 1035-1038 (1995); Hurle and Gross, Curr. Op. Biotech. 5: 428-433 (1994); and U.S. Patent No. 6,982,321 and Ditto 7,087,409.

A "human antibody" is an antibody having an amino acid sequence corresponding to an amino acid sequence of an antibody produced by humans and/or an antibody produced by using any of the techniques for producing human antibodies disclosed herein. This definition of the human antibody specifically excludes humanized antibodies comprising non-human antigen binding residues. The human antibody can be produced using a variety of techniques known in this field of the art, including phage display libraries. Hoogenboom and Winter, J. Mol. Biol., 227: 381 (1991); Marks et al., J. Mol. Biol., 222: 581 (1991). Further available for the preparation of a human monoclonal antibody is methods described in Cole et al., Monoclonal Antibodies and Cancer Therapy, Alan R. Liss, p. 77 (1985); and Boerner et al., J. Immunol., 147(1): 86-95 (1991). Also refer to van Dijk and van de Winkel, Curr. Opin. Pharmacol., 5: 368-74 (2001). Human antibodies have been modified to produce such antibodies in response to antigen administration, but it can be produced by administering an antigen to transgenic animals whose endogenous loci are not functional, for example, immunized xeno-mouse (see U.S. Patent No. 6,075,181 and Ditto 6,150,584 regarding XENOMOUSE^{™} technology). For example, also see Li et al., Proc. Natl. Acad. Sci. USA, 103: 3557-3562 (2006) regarding a human antibody produced via human B cell hybridoma technology.

When used in the present specification, the terms "high frequency variable region," "HVR" or "HV" refer to a region of an antibody variable domain whose sequence is highly variable and/or forms a structurally defined loop. In general, an antibody contains six HVRs, three (H1, H2, H3) in the VH and three (L1, L2, L3) in the VL. In a natural antibody, H3 and L3 represent the greatest diversity of six HVRs, in particular, H3 is thought to play a unique role in conferring good specificity to the antibody. For example, see Xu et al., Immunity 13: 37-45 (2000); Johnson and Wu, in Methods in Molecular Biology 248: 1-25 (Lo, ed., Human Press, Totowa, NJ, 2003). In fact, a naturally occurring camel antibody constituted by heavy chains alone is functional and stable in the absence of light chains. For example, see Hamers-Casterman et al., Nature 363: 446-448 (1993); and Sheriff et al., Nature Struct. Biol. 3: 733-736 (1996).

Numerous depictions of HVRs are used and are included herein. Kabat complementarity-determining regions (CDR) are based on sequence variation and are the most commonly used (Kabat et al., Sequences of Proteins of Immunological Interest, 5th edition, Public Health Service, National Institutes of Health, Bethesda, MD. (1991)). Chothia instead refers to the position of the structural loops (Chothia and Lesk J. Mol. Biol. 196: 901-917 (1987)). AbM HVR represents a compromise between the Kabat HVR and Chothia structural loops and is used by Oxford Molecular's AbM antibody modeling software. "Contact" HVR is based on analysis of the available complex crystal structures.

HVR can contain the following "expanded HVR": VL's 24-36 or 24-34 (L1), 46-56 or 50-56 (L2) and 89-97 or 89-96 (L3) and VH's 26-35 (H1), 50-65 or 49-65 (H2) and 93-102, 94-102, or 95-102 (H3). Numbers are applied to the variable domain residues in accordance with the above such as Kabat, etc., to define each of these.

The expression "variable domain residue numbering by Kabat" or "amino acid position numbering as described in Kabat" and its different phrases refer to the numbering system used for the light chain variable domains or heavy chain variable domains in the compilation of antibodies such as Kabat described above. When this numbering system is used, the actual linear amino acid sequence is reduced or increased by two or three amino acids contained, corresponding to the shortening of FRs or HVRs in the variable domains or their insertion into them. For example, a heavy chain variable domain can contain a residue inserted after heavy chain FR residue 82 (for example, residues 82a, 82b and 82c, etc., by Kabat) and a single amino acid insertion after residue 52 of H2 (residue 52a by Kabat). The Kabat numbering of the residue may be determined for a given antibody by aligning it with homologous regions of the sequence of the antibody using a "standard" Kabat numbering sequence.

A "framework" or "FR" residue is a variable domain residue other than an HVR residue defined in the present specification.

The antibody may be a conjugate antibody bonded to various kinds of molecules such as polyethylene glycol (PEG), radioactive substances, toxins, etc. Such a conjugate antibody can be obtained by chemically modifying the resulting antibody. Incidentally, the modifying method of an antibody have already been established in this field. In the "antibody" in the present specification, these conjugate antibodies are included.

In the antibody, not only a bivalent antibody represented by IgG, but also a monovalent antibody, or a multivalent antibody represented by IgM are also contained. In the multivalent antibody of the present disclosure, a multivalent antibody having entirely the same antigen binding site or a multivalent antibody having partially or entirely different antigen binding site is contained.

Further, the antibody may be a bispecific antibody. The bispecific antibody is an antibody that has variable regions that recognize different epitopes in the same antibody molecule, which epitopes may be present in different molecules or may be present in the same molecule.

A method for producing a bispecific antibody is well known. For example, a bispecific antibody can be produced by combining two kinds of antibodies with different recognition antigens. The antibody to be combined may be one-half molecule of an antibody each having an H chain and an L chain, or one-quarter molecule of an antibody consisting only of an H chain. Alternatively, a bispecific antibody producing hybrid cells can be produced by fusing hybridomas that produce different monoclonal antibodies. Further, a bispecific antibody can be produced by genetic engineering means.

The antibody may be a low molecular weight antibody. The low molecular weight antibody contains antibody fragments in which a portion of the full-length antibody is missing. As long as it binds to PD-L1, partial deletion of the antibody molecule is acceptable. The antibody fragments in the present disclosure preferably contain either or both of the heavy chain variable region (VH) and the light chain variable region (VL). The amino acid sequence of VH or VL can include additions, deletions and/or substitutions. Further, as long as it binds to PD-L1, either or both of VH and VL can be deleted. In addition, the antibody fragments may also be chimeric or humanized. Specific examples of the antibody fragments may be mentioned, for example, Fab, Fab', F(ab')2, Fv, etc. Also, specific examples of the low molecular weight antibody may be mentioned, for example, Fab, Fab', F(ab')2, Fv, scFv, diabody, sc(Fv)2, etc.

As a material acceptable for formulation, there may be mentioned, for example, sterile water or physiological saline, stabilizers, excipients, buffers, preservatives, surfactants, chelating agents (EDTA, etc.), binding agents, etc.

As the surfactant, there may be mentioned a nonionic surfactant, and there may be mentioned, for example, sorbitan fatty acid esters such as sorbitan monocaprylate, sorbitan monolaurate, sorbitan monopalmitate, etc.; glycerol fatty acid esters such as glycerol monocaprylate, glycerol monomyritate, glycerol monostearate, etc., having HLB of 6 to 18; etc., as typical examples.

In addition, as the surfactant, there may be also mentioned an anionic surfactant, and may be mentioned, for example, alkyl sulfates having an alkyl group of 10 to 18 carbon atoms, such as sodium acetyl sulfate, sodium lauryl sulfate, sodium oleyl sulfate, etc.; polyoxyethylene alkyl ether sulfates having an average added molar number of ethylene oxide of 2 to 4 and a number of carbon atoms of an alkyl group of 10 to 18, such as sodium polyoxyethylene lauryl sulfate, etc.; sodium alkyl sulfosuccinates having an alkyl group of 8 to 18 carbon atoms, such as sodium lauryl sulfosuccinate, etc.; natural surfactants, for example, lecithin, glycerophospholipids; sphingophospholipids such as sphingomyelin, etc.; and sucrose fatty acid esters having a fatty acid of 12 to 18 carbon atoms etc., as typical examples..

As the buffer, there may be mentioned phosphoric acid, citrate buffer, acetic acid, malic acid, tartaric acid, succinic acid, lactic acid, potassium phosphate, gluconic acid, caprylic acid, deoxycholic acid, salicylic acid, triethanolamine, fumaric acid, etc., other organic acids, etc., or carbonate buffer, Tris buffer, histidine buffer (for example, L-histidine and glacial acetic acid), imidazole buffer, etc.

In addition, a solution formulation may also be prepared by dissolving in an aqueous buffer solution known in the field of solution formulations. A concentration of the buffer solution is generally from 1 to 500 mM, preferably from 5 to 100 mM, and more preferably from 10 to 20 mM.

As sugars and carbohydrates such as polysaccharides and monosaccharides, etc., there may be mentioned, for example, dextran, glucose, fructose, lactose, xylose, mannose, maltose, sucrose (refined white sugar, etc.), trehalose (trehalose hydrate, etc.), raffinose, etc.

As sugar alcohols, there may be mentioned, for example, mannitol, sorbitol, inositol, etc.

When it is made an aqueous solution for injection, there may be mentioned, for example, isotonic solutions containing, for example, physiological saline, dextrose or other auxiliary agents, such as D-sorbitol, D-mannose, D-mannitol and sodium chloride, and it may be used in combination with appropriate dissolution aids, such as alcohol (ethanol, etc.), polyalcohol (propylene glycol, PEG, etc.), nonionic surfactants (polysorbate 20, polysorbate 80, HCO-50), etc.

If desired, it may further contain diluents, dissolution aids, pH adjusters, painless agents, sulfur-containing reducing agents, antioxidants, etc. It may contain sodium dihydrogen phosphate monohydrate, anhydrous sodium monohydrogen phosphate, sodium edetate hydrate and sodium hydroxide.

One of the embodiments of the present disclosure is a pharmaceutical composition comprising an anti-PD-Ll antibody as an effective ingredient, which comprises using paclitaxel and an anti-VEGF antibody in combination.

One of the embodiments of the present disclosure is a pharmaceutical composition comprising an anti-VEGF antibody as an effective ingredient, which comprises using paclitaxel and an anti-PD-L1 antibody in combination.

One of the embodiments of the present disclosure is a pharmaceutical composition containing paclitaxel as an effective ingredient, which comprises using an anti-PD-Ll antibody and an anti-VEGF antibody in combination.

One of the embodiments of the present disclosure is a pharmaceutical composition which comprises paclitaxel, an anti-PD-Ll antibody and an anti-VEGF antibody.

One of the embodiments of the present disclosure is a pharmaceutical composition which comprises paclitaxel, an anti-VEGF antibody and an anti-PD-Ll antibody in combination.

The pharmaceutical composition of the present disclosure can be used for the treatment of cancer. The cancer is selected from the group consisting of ovarian cancer, non-small cell lung cancer, breast cancer, gastric cancer, uterine cancer, head and neck cancer with recurrence or distant metastasis, esophageal cancer with recurrence or distant metastasis, angiosarcoma, progression or recurrent cervical cancer, and recurrent or refractory germ cell tumor (testicular tumor, ovarian tumor, extragonadal tumor). One of the embodiments of the present disclosure is that the cancer is breast cancer. One of the embodiments of the present disclosure is that the cancer is estrogen receptor-positive HER2-negative breast cancer.

The pharmaceutical composition according to the present disclosure can be made as a single formulation (combination product) or two or more formulations obtained by formulating these effective ingredients separately. The above-mentioned formulations can be made into tablets, granules, powder, capsules, emulsions, suspensions, syrups, or injectables such as sterile solutions and suspensions according to conventionally carried means. When these active ingredients are separately formulated into two or more kinds of formulations, the individual formulations can be administered simultaneously or at regular time intervals. Said two or more kinds of these preparations may be administered at different number of times per day. The pharmaceutical composition according to the present disclosure can be administered systemically or topically, orally or parenterally. When these effective ingredients are separately formulated into two or more kinds of formulations, the individual formulations can also be administered by different pathway.

When the pharmaceutical composition according to the present disclosure is made in two kinds of different formulations, they are likely to be administered at the same time or at very short intervals, and therefore, for example, the accompanying documents or sales brochures of the commercially available pharmaceuticals can state that each is to be used together. In addition, it can be made a kit comprising formulations containing each of them.

The dosage of the medicament of the present disclosure varies depending on the subject of administration, administration method, etc., and, for example, paclitaxel can be administered by intravenous infusion at a dosage of 90 mg/m² (body surface area) or more, 100 mg/m² or more, 210 mg/m² or more, and 1,000 mg/m² once a day. Also, the anti-PD-Ll antibody can be administered, for example, at a dose of 1 mg or more once, 840 mg or less, 1,000 mg or less, or 1,200 mg or less, or at a dose of 1.0 mg/kg (body weight) or more, 2.5 mg/kg (body weight) or more, 10 mg/kg (body weight) or more, 20 mg/kg (body weight) or more, and 100 mg/kg (body weight) or less once. The anti-VEGF antibody can be administered, for example, 5 mg/kg (body weight) or more, 10 mg/kg (body weight) or more, 15 mg/kg (body weight) or more, and 100 mg/kg (body weight) or less once.

In one embodiment of the present disclosure, the dosages of the medicament of the present disclosure include, for example, paclitaxel at 90 mg/m² (body surface area) once by intravenous infusion, the anti-PD-L1 antibody (for example, atezolizumab) at a dosage of, for example, 840 mg/body once by intravenous infusion, and the anti-VEGF antibody (for example, bevacizumab) is administered, for example, at 10 mg/kg (body weight) by intravenous infusion.

In one embodiment of the present disclosure, the medicament of the present disclosure may be administered, for example, on days 1, 8 and 15 of each 28-day cycle, paclitaxel is administered at a dose of 90 mg/m² (body surface area) once by intravenous infusion, on days 1 and 15, the anti-PD-Ll antibody (for example, atezolizumab) is administered at a dose of 840 mg/body once by intravenous infusion, and on days 1 and 15, the anti-VEGF antibody (for example, bevacizumab) is administered at a dose of 10 mg/kg (body weight) by intravenous infusion.

In one embodiment of the present disclosure, the treatment includes an induction phase and maintenance phase (or "maintenance therapy"). In a certain embodiment, the induction phase includes administration of the anti-PD-Ll antibody (for example, atezolizumab) at a dose of 840 mg, administration of paclitaxel at a dose of 90 mg/m² (body surface area), and administration of the anti-VEGF antibody (for example, bevacizumab) at a dose of 10 mg/kg (body weight) on day 1 of each 28-day cycle. In one embodiment of the present disclosure, the maintenance phase includes administration of the PD-1 axis binding antagonist anti-PD-L1 antibody (for example, atezolizumab) at a dose of 840 mg, and administration of the anti-VEGF antibody (for example, bevacizumab) at a dose of 10 mg/kg (body weight), including administration unless exacerbation is observed after discontinuation of paclitaxel or criteria for discontinuation of therapy are not met.

In one embodiment of the present disclosure, it is a method of treating or preventing cancer characterized by the use of an anti-PD-Ll antibody, paclitaxel and an anti-VEGF antibody in combination. The anti-PD-L1 antibody, the anti-VEGF antibody and paclitaxel can be administered to the patient simultaneously or sequentially.

In one embodiment of the present disclosure, "use in combination" contemplated above includes administration using separate formulations or a single medicament preparation simultaneously, and administration including successive administration in any order, preferably with a period of time during which both (or all) effective agents exert biologically active at the same time. Formulations and schedules of dosing for such chemotherapeutic agents may be determined and used according to the manufacturer's instructions or empirically by those skilled in the art.

In the present specification, "advanced recurrent breast cancer" refers to any of unresectable locally advanced breast cancer, recurrent breast cancer, and Stage IV breast cancer. "Locally advanced breast cancer" refers to the cases where cancer has spread to the skin on the surface of the breast or chest wall, or in the case of inflammatory breast cancer, cases in which metastasis has spread to the supraclavicular lymph nodes, and the disease stage corresponds to stage IIIB or IIIC. Locally advanced breast cancer is more likely to be accompanied by micrometastases (small metastases not visible to the eye) anywhere in the body.

In the present specification, "recurrence" refers to the phenomenon that invisible masses of cancer cells lurking somewhere in the body as micrometastases from the first time breast cancer occurred, and appears after undergoing surgery after evading initial treatment. When it occurs in the breast or surrounding skin or lymph nodes of the breast at the operated side, it is called "local recurrence", and when it occurs in places distant from the breast, such as bones or lungs, it is called "metastasis" or "distant metastasis". It may be accompanied by some symptoms (constant pain in a certain place, cough that does not go away, etc.), or it may be completely asymptomatic. In one embodiment of the present disclosure, the individual has recurrent breast cancer. In one embodiment of the present disclosure, the individual may also have reduced symptoms associated with recurrent breast cancer.

In one embodiment of the present disclosure, an individual has hormone resistance (recurrent within 2 years of initiation of postoperative endocrine therapy, or progression within 6 months of endocrine therapy for advanced recurrent breast cancer is admitted) or Life threatening metastases (state with symptomatic metastases such as multiple liver metastases, lung metastases, carcinomatous pleuritis, and carcinomatous lymphangiosis, which require symptomatic relief through rapid tumor reduction).

In some embodiments, the individual is Asian. In some embodiments, the individual is of Asian descent.

In some embodiments, an individual is "high PD-L1". In some embodiments, a patient is "high PD-L1" when the tumor cells expressing PD-L1 in a pre-treatment sample derived from the patient exceed a total of 50% of all tumor cells in the sample. In some embodiments, a patient is "high PD-L1" when the percentage of the tumor-infiltrating immune cells expressing PD-L1 in a pre-treatment sample derived from the patient exceeds 10% of the total tumor immune cells in the tumor region in the sample. In some embodiments, PD-L1 expression exceeding 10% of tumor-infiltrating immune cells in a pre-treatment sample is defined/scored as "IC3". In some embodiments, the pre-treatment sample is a fresh tumor sample. In some embodiments, the pre-treatment sample is a formalin-fixed paraffin-embedded (FFPE) tumor sample. In some embodiments, PD-L1 expression levels in the tumor cells and/or tumor-infiltrating immune cells in the pre-treatment sample are determined by immunohistochemical assay. In some embodiments, the immunohistochemical assay is VENTANA SP142 assay.

In some embodiments, a patient is "low PD-L1" when the tumor cells expressing PD-L1 in a pre-treatment sample derived from the patient are 1% to less than 5% in total of all tumor cells in the sample. In some embodiments, a patient is "low PD-L1" when the tumor cells expressing PD-L1 in the pre-treatment sample derived from the patient are 5% to less than 50% in total of the tumor immune cells in the tumor region in the sample. In some embodiments, a patient is "low PD-L1" when the tumor-infiltrating immune cells expressing PD-L1 in the pre-treatment sample derived from the patient are 1% to less than 5% in total of all tumor-filtrating immune cells in the sample. In some embodiments, PD-L1 expression in 1% to less than 5% of tumor-infiltrating immune cells in the pre-treatment sample is defined/scored as "IC1". In some embodiments, a patient is "low PD-L1" when the tumor-infiltrating immune cells expressing PD-L1 in the pre-treatment sample derived from the patient are 5% to less than 10% in total of all tumor-filtrating immune cells in the sample. In some embodiments, PD-L1 expression in 5% to less than 10% of the tumor-infiltrating immune cells in the pre-treatment sample is defined/scored as "IC2". In some embodiments, the pre-treatment sample is a fresh tumor sample. In some embodiments, pre-treatment sample is a formalin-fixed paraffin-embedded (FFPE) tumor sample. In some embodiments, PD-L1 expression levels in the tumor cells and/or tumor-infiltrating immune cells in the pre-treatment sample are determined by immunohistochemical assay. In some embodiments, the immunohistochemical assay is VENTANA SP142 assay.

In some embodiments, an individual is "PD-L1 negative". In some embodiments, a patient is "PD-L1 negative" when the tumor cells expressing PD-L1 in the pre-treatment sample derived from the patient is less than 1% in total of the tumor immune cells in the tumor region in the sample. In some embodiments, a patient is "PD-L1 negative" when the tumor-infiltrating immune cells expressing PD-L1 in the pre-treatment sample derived from the patient is less than 1% in total of all tumor-filtrating immune cells in the sample. In some embodiments, PD-L1 expression in less than 1% of the tumor-infiltrating immune cells in the pre-treatment sample is defined as "IC0". In some embodiments, the pre-treatment sample is a fresh tumor sample. In some embodiments, the pre-treatment sample is a formalin-fixed paraffin-embedded (FFPE) tumor sample. In some embodiments, PD-L1 expression levels in tumor cells and/or tumor-infiltrating immune cells in the pre-treatment sample is determined by immunohistochemical assay. In some embodiments, the immunohistochemical assay is VENTANA SP142 assay.

In some embodiments, IC0, IC1, IC2, and IC3 are defined/scored as outlined in the following table:
Definitions of scoring of exemplary tumor-infiltrating immune cells (immune cell: IC)

**[Table 1]**

| **PD-L1 IC dying standard*** | |
|---|---|
| **IC score** | **Definition** |
| IC0 | less than 1% |
| IC1 | 1% or more and less than 5% |
| IC2 | 5% or more and less than 10% |
| IC3 | 10% or more |

### [Example 1]

Hereinafter, the present disclosure will be explained specifically by Examples, but the present disclosure is not limited to these Examples.

Anti-tumor activity using anti-PD-L1 antibody (10F. 9G2), anti-VEGF antibody (B20-4.1.1) and paclitaxel in combination in a syngeneic mouse model using mouse breast cancer cell line FM3A

Anti-PD-L1 antibody (10F.9G2) was purchased from BioLegend and, paclitaxel was purchased from Wako Pure Chemicals Co. Anti-VEGF antibody (B20-4.1.1) was provided by Genentech, Inc. FM3A cells were cultured using cell culture flasks in an incubator (set at 37°C and 5% CO2). Cells were collected, resuspended at 5×10⁶ cells/mL, and inoculated subcutaneously (1×10⁶ cells/mouse) at 200 µL/mouse into the right abdomen of each C3H/HeN mouse (Charles River Japan, Inc.). Once a palpable tumor was established, animals were randomized to test groups so that each group had a similar mean tumor volume at the start of the study. The randomization date was set as the start of medicament administration, 100 mg/kg of paclitaxel was administered once weekly into the tail vein, 5 mg/kg of 10F.9G2 was administered twice weekly intraperitoneally, and 10 mg/kg of B20-4.1.1 was administered once weekly intraperitoneally. As a solvent control for paclitaxel, paclitaxel medium (equal volumes of ethanol and Cremophor (Registered Trademark) EL are mixed and diluted 10-fold with physiological saline) was administered (group constitution is shown in Table 1).

**[Table 2]**

| Group | Administered substance | Administration dose (mg/kg) | Administration route | Administration frequency |
|---|---|---|---|---|
| PTX medium control group | PTX medium | 0 | i. v. | Once/week |
| Anti-PD-L1 antibody group | Anti-PD-L1 antibody | 5 | i. p. | Twice/week |
| | PTX medium | 0 | i. v. | Once/week |
| Anti-VEGF antibody group | Anti-VEGF antibody | 10 | i. p. | Once/week |
| | PTX medium | 0 | i. v. | Once/week |
| PTX group | PTX | 100 | i. v. | Once/week |
| PTX + Anti-PD-L1 antibody group | PTX | 100 | i. v. | Once/week |
| | Anti-PD-L1 antibody | 5 | i. p. | Twice/week |
| PTX + Anti-VEGF antibody group | PTX | 100 | i. v. | Once/week |
| | Anti-VEGF antibody | 10 | i. p. | Once/week |
| PTX + Anti PD-L1 antibody + Anti-VEGF antibody group | PTX | 100 | i . v. | Once/week |
| | Anti-PD-L1 antibody | 5 | i . p. | Twice/week |
| | Anti-VEGF antibody | 10 | i . p. | Once/week |

Tumor growth was recorded over time by measuring the long diameter (mm) and short diameter (mm) of the tumor with a caliper. Tumor volume was calculated using the following calculation formula: Tumor volume (mm3) = long diameter (mm) × short diameter (mm) × short diameter (mm) × 0.5

In Fig. 1, the average tumor volumes for each group are shown. In Fig. 2, the individual data on day 20 are shown by each group. The average tumor volume of the combination group of paclitaxel + anti-PD-Ll antibody + anti-VEGF antibody was the lowest, and some individuals showed almost no tumor growth. Also, even when compared to the combination group of paclitaxel + anti-VEGF antibody, the average tumor volume of the combination group of paclitaxel + anti-PD-Ll antibody + anti-VEGF antibody was lower.

Incidentally, the fact that FM3A is hormone receptor positive is described in the following Web site and paper. https://www. sigmaaldrich.com/JP/ja/technical-documents/technical-article/cell-culture-and-cell-culture-analysis/primary-cell-culture/breast-cancer-cell-lines https://pubmed.ncbi.nlm.nih.gov/23803037/
https://pubmed.ncbi.nlm.nih.gov/24344011/

### [Example 2]

Randomized, controlled, open-label, real medication (treatment), parallel-group, phase III study of paclitaxel + bevacizumab + atezolizumab for hormone receptor positive HER2 negative advanced recurrent breast cancer (AMBITION test (JCOG1919E), NCT04732598)

The above-mentioned test data is to evaluate the efficacy (superiority in progression-free survival period) and safety of adding atezolizumab to paclitaxel + bevacizumab therapy in hormone receptor positive HER2 negative advanced recurrent breast cancer, and demonstrate that these combination collectively provides a meaningful clinical benefit to cancer patients.

Patients who meet all of the following eligibility criteria and none of the exclusion criteria will be considered eligible for enrollment.

### Main selection criteria

1) Histologically diagnosed with breast cancer (invasive cancer).
2) Histologically diagnosed with hormone receptor positive (at least one of ER and PgR is positive) and HER2 negative. However, when there are multiple specimens, the histological test results of the most recent specimen among the specimens that satisfy the eligibility criteria 1) and 2) shall be used.
3) Diagnosed with advanced recurrent breast cancer (any of unresectable locally advanced breast cancer, recurrent breast cancer and Stage IV breast cancer).
4) Age on the date of enrollment is 20 years or older. Male or female is not questioned.
5) ECOG Performance status (PS) is 0 to 2.
6) Have measurable disease.
7) Have hormone refractory 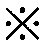 1, or Life threatening metastasis 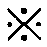 2.
   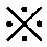 1: Hormone refractory: Recurrence within 2 years after the start of postoperative endocrine therapy, or progression within 6 months of endocrine therapy for advanced recurrent breast cancer is admitted.
   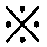 2: Life threatening metastasis: A state in which symptomatic metastasis exists and symptoms palliation is required by prompt tumor shrinkage. Examples include multiple liver metastases, lung metastases, cancerous pleurisy, cancerous lymphangitis, etc.
8) The PD-L1 expression status (IC0, IC1, IC2, IC3) of the tumor has been confirmed by a central measuring institution.
9) Having no active brain metastasis which requirs treatment.
10) No history of prior chemotherapy treatment for advanced recurrent breast cancer. However, a history of preoperative/postoperative chemotherapy including paclitaxel or docetaxel is acceptable if at least 6 months have elapsed since the last dose.
11) The most recent laboratory values within 14 days prior to enrollment (the same day of the week two weeks prior to the date of enrollment is acceptable) satisfy all of the following.
   (a) Number of neutrophil ≧ 1,500/mm^3
   (b) Hemoglobin ≧ 9.0 g/dL (no blood transfusion within 14 days prior to blood collection for the test used for registration)
   (c) Number of platelet ≧ 10 × 104/mm^3
   (d) Total bilirubin ≦ 1.5 mg/dL
   (e) AST ≦ 100 IU/L (≦ 150 IU/L in case of liver metastasis)
   (f) ALT ≦ 100 IU/L (≦ 150 IU/L in case of liver metastasis)
   (g) Serum creatinine ≦ 1. 2 mg/dL
   (h) PT-INR ≦ 1.5 provided that when prophylactic anticoagulants such as warfarin are being taken, then PT-INR ≦ 3. 0
   (i) Urine protein (test paper method) is 1+ or less
12) For women of childbearing potential, consent to contraception from the time of obtaining consent until at least 6 months after completion of protocol treatment. For lactating patients, the patient agrees not to breastfeed from the start of protocol treatment until at least 6 months after the end of protocol treatment. For men, he agrees to contraception from the start of protocol treatment until at least 6 months after the end of protocol treatment.

### Main exclusion criteria

1) Patients have active overlapping cancer. However, the following (a) to (c) are excluded: (a) Completely resected following cancer: basal cell carcinoma, Stage I spinous cell carcinoma, intraepithelial carcinoma, intramucosal carcinoma, superficial bladder carcinoma, (b) gastrointestinal cancer curatively resected by ESD or EMR, (c) other cancer that has not recurred for at least 5 years.
2) Patients have infectious disease requiring systemic treatment.
3) Complicated with active peptic ulcer.
4) Patients have hypertension that is poorly controlled (patients cannot control to systolic blood pressure ≦ 150 mmHg and diastolic blood pressure ≦ 100 mmHg) even when two or more kinds of antihypertensive drugs are used.
5) Patients have symptomatic congestive heart failure, unstable angina pectoris, or arrhythmia requiring treatment at the time of enrollment.
6) Patients have a history of myocardial infarction within 1 year prior to enrollment.
7) Patients have undergone major surgery or incisional biopsy or sustained significant trauma within 28 days prior to enrollment. CV port placement is not deemed to be major surgery.
8) Patients have deep vein thrombosis or pulmonary embolism at the time of enrollment or have a history of it within 1 year prior to enrollment.
9) Patients have used anticoagulants within 10 days prior to enrollment. However, patients who take anticoagulants for prophylaxis at a stable dose are eligible for enrollment.
10) Patients have a history of idiopathic pulmonary fibrosis, organizing pneumonia (bronchiolitis obliterans, etc.), drug-induced pneumonitis or idiopathic pneumonitis. However, patients with a history of drug-induced pneumonitis who are asymptomatic at the time of enrollment may be enrolled if they undergo periodic chest X-ray examinations and careful follow-up, including auscultation and interview.
11) Chest CT shows findings of active pneumonitis. However, a history of localized radiation pneumonitis (fibrosis) within the irradiated field is excluded.
12) There is a history of administration of investigational drug atezolizumab or immune checkpoint inhibitors (anti-PD-1, anti-PD-Ll, anti-CTLA-4 antibody drugs, etc.) or immunostimulants (example: interferon, interleukin-2).
13) Active autoimmune disease or immunodeficiency or a history thereof (example: myasthenia gravis, myositis, autoimmune hepatitis, systemic lupus erythematosus, rheumatoid arthritis, inflammatory bowel disease, antiphospholipid antibody syndrome, Wegener's granulomatosis, Sjogren's syndrome, Guillain-Barre syndrome, multiple sclerosis, etc.). However, the following are excluded.
   - Patients with autoimmune hypothyroidism who are using stable doses of thyroid hormone preparations.
   - Patients with eczema, psoriasis, chronic lichen simplex, or vitiligo vulgaris whose symptoms are limited to the skin only, and whose rash is less than 10% of the body surface area, and are well controlled with low-titer topical corticosteroids alone.
   - Patients who have not received acute exacerbation of underlying disease requiring psoralen and ultraviolet A therapy, methotrexate, retinoids, biologics, oral calcineurin inhibitors, high-titer or oral adrenocortical steroids within the past 12 months
14) Patients who have received a live attenuated vaccine within 4 weeks prior to enrollment or are expected to require a live attenuated vaccine within 5 months of completion of protocol treatment.
15) Patients have not recovered from clinically significant toxicity developed with prior therapy, with the exception of alopecia and Grade 1 peripheral neuropathy.
16) Patients have hypersensitivity or contraindication to any component of the treatment, including macrogol glycerol ricinoleate (Cremophor (Registered Trademark)), etc., which is an additive to paclitaxel.
17) Patients are positive for any of HIV antibody, HBs antigen or HCV antibody (however, even if HCV antibody is positive, cases where HCV-RNA is not detected are not excluded).
18) The patients are negative for HBs antigen, positive for HBs antibody or HBc antibody, and HBV-DNA quantification is 20 IU/mL (1.3 log IU/mL) or more.
19) Women who are pregnant, lactating or possibly pregnant.
20) Patients who are complicated by psychosis or psychiatric symptoms that interfere with daily life and are judged to be difficult to participate in the study.

### Treatment group

### Group A: paclitaxel +bevacizumab therapy

The following regimen will be administered once every 28 days, and treatment continued as long as no progression is observed or criteria for discontinuation of treatment are met.

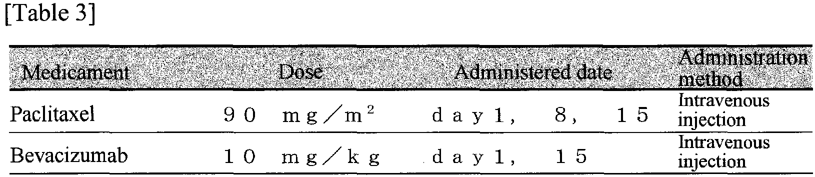

### Group B: paclitaxel +bevacizumab +atezolizumab therapy

The following regimen will be administered once every 28 days, and treatment continued as long as no progression is observed or criteria for discontinuation of treatment are met.

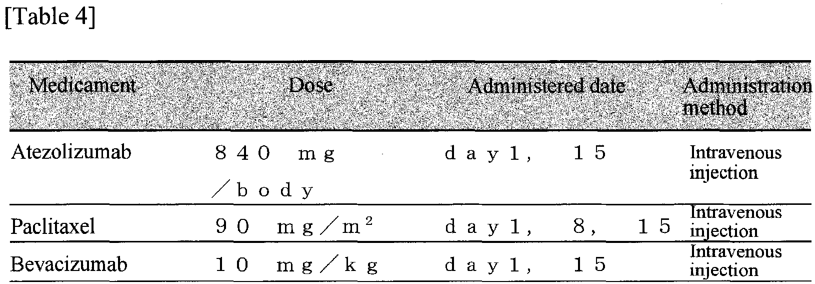

Each treatment cycle is 28 days, with Group A being the combined group of paclitaxel and bevacizumab and Group B being the combined group of paclitaxel, bevacizumab and atezolizumab.

Patients receive intravenous atezolizumab (fixed dose of 840 mg), bevacizumab (10 mg/kg) and paclitaxel (90 mg/m²) on day 1 of each cycle. Thereafter, on days 1 and 15 of each cycle, atezolizumab (fixed dose of 840 mg) and bevacizumab (10 mg/kg) are administered intravenously, and on days 1, 8 and 15 of each cycle, paclitaxel (90 mg/m²) is administered intravenously.

Patients are treated until clinical benefit is lost, unacceptable toxicity occurs, or consent is withdrawn. If one of the treatments is stopped completely, treatment with only the other drug may be continued.

### Primary endpoints

### Progression-free survival period (institutional determination)

### Secondary endpoints

### Progression-free survival period (central determination)

Overall survival, response rate (institutional determination)
Duration of response (institutional determination)
Response rate (central determination)
Duration of response (central determination)
Adverse event rate
Serious adverse event rate
Immune-related adverse event rate
Progression-free survival period in PD-L1 positive subpopulation (institutional determination)
Progression-free survival period in PD-L1 positive subpopulation (central determination)
Overall survival in PD-L1 positive subpopulation
Response rate in PD-L1 positive subpopulation (institutional determination)
Response rate in PD-L1 positive subpopulation (central determination)

Imaging studies (CT or MRI) for tumor evaluation will be performed every 8 weeks up to week 24 and every 12 weeks after week 25, starting from the start of protocol treatment, without any change even if the start of the course is postponed.

Judgment of tumor shrinkage efficacy will be carried out according to the procedure in accordance with the "New response evaluation criteria in solid tumours: Revised (RECIST guideline) version 1.1-Japanese translation JCOG version-: Revised RECIST guideline for the determination of treatment response in solid tumors (JCOG version) (version 1.1) 43".

### Efficacy criteria for target lesion

### · CR (Complete Response): completely effective

All non-lymph node target lesions have disappeared and the short diameter of all lymph node target lesions is less than 10 mm. If lymph node target lesions are selected at baseline, the effect of the target lesions may be CR even if the sum of diameters does not reach 0 mm

### · PR (Partial Response): partially effective

The sum of diameters of target lesions compared to the baseline sum of diameters is 30% or more.

### · PD (Progressive Disease): progression

When the sum of diameters of the target lesion increases by 20% or more compared to the minimum sum of diameters during the course of the disease (if the baseline is the minimum value of diameters during the course of the disease, this is the minimum sum of diameters), and the sum of diameters increases by 5 mm or more in absolute value.

### · SD (Stable Disease): Stable

When there is no reduction corresponding to PR and no increase corresponding to PD.

### · NE (Not all Evaluated): Evaluation is deficient.

When the test cannot be performed for some reason, or when neither CR, PR, PD nor SD can be determined.

In the present specification, judgement of PD-L1 positivity/negativity is carried out by immunohistochemical detecting programmed cell death ligand-1 (PD-L1) protein in tissues and cells, for example, by the in vitro diagnostic drug Ventana OptiView PD-L1 (SP142) (https://www.pmda.go.jp/review-services/drug-reviews/review-information/cd/0001.html).

### Results

Patient enrollment began in January 2021, and as of November 2021, approximately 90 patients had been treated at each dose cohort level of the Group A and Group B.

Patients in the study underwent imaging studies (CT or MRI) for tumor evaluation at 8 weeks from the start of protocol treatment and every 8 weeks thereafter.

As of November 2021, at current dose levels (paclitaxel (90 mg/m², every week for 3 weeks + 1 week rest) + bevacizumab (10 mg/kg, every 2 weeks; Q2W) + atezolizumab (fixed dose of 840 mg, every 2 weeks; Q2W)), no apparent safety have been identified for the continuation of the study.

Any one or more of the primary, secondary or additional endpoints are achieved, while obtaining acceptable toxicity according to the safety endpoints specified herein. In addition, superior efficacy and or safety is achieved by adding atezolizumab to paclitaxel + bevacizumab therapy as compared to the combination therapy of bevacizumab and paclitaxel.

### UTILIZABILITY IN INDUSTRY

The present disclosure is useful for the treatment of hormone receptor positive HER2 negative advanced recurrent breast cancer.

### SEQUENCE ID LISTING

FP4848PCT_ST.25TXT

## Claims

1. A pharmaceutical composition which comprises a composition for use in combination with paclitaxel and an anti-VEGF antibody to treat estrogen receptor-positive HER2-negative breast cancer or to delay progression of the cancer in an individual, which comprises an anti-PD-Ll antibody as an effective ingredient.

2. The pharmaceutical composition according to Claim 1, wherein the anti-PD-Ll antibody is atezolizumab.

3. The pharmaceutical composition according to any of Claims 1 to 2, wherein the anti-VEGF antibody is bevacizumab, ramucirumab or aflibercept beta.

4. The pharmaceutical composition according to any of Claims 1 to 3, wherein the anti-VEGF antibody is bevacizumab.

5. The pharmaceutical composition according to any of Claims 1 to 4, wherein the breast cancer is hormone receptor-positive HER2-negative breast cancer.

6. The method for treating estrogen receptor-positive HER2-negative breast cancer or delaying progression of the cancer, which comprises administering an anti-PD-Ll antibody, an anti-VEGF antibody and paclitaxel in combination.

7. The method according to Claim 6, wherein the anti-PD-Ll antibody is atezolizumab.

8. The method according to any of Claims 6 to 7, wherein the anti-VEGF antibody is bevacizumab, ramucirumab or aflibercept beta.

9. The method according to any of Claims 6 to 8, wherein the anti-VEGF antibody is bevacizumab.

10. The method according to any of Claims 6 to 9, wherein the breast cancer is hormone receptor-positive HER2-negative breast cancer.

11. A medicament comprising atezolizumab for treating or delaying progression of estrogen receptor-positive HER2-negative breast cancer in an individual for use in combination with paclitaxel and anti-VEGF antibody.

12. The medicament according to Claim 11, wherein the breast cancer is hormone receptor-positive HER2-negative breast cancer.

13. A method for treating estrogen receptor-positive HER2-negative breast cancer which comprises using atezolizumab, an anti-VEGF antibody and paclitaxel in combination.

14. The treating method according to Claim 13, wherein the breast cancer is hormone receptor-positive HER2-negative breast cancer.
